# EUROPÄISCHE PATENTSCHRIFT

(11) **EP 0 860 168 B1**
(45) Veröffentlichungstag und Bekanntmachung des Hinweises auf die Patenterteilung: **08.08.2007**
(21) Anmeldenummer: 98102196.7
(22) Anmeldetag: 09.02.1998
(51) Int. Cl.: A61K 31/35, A61K 31/47

(54) **Verwendung von Agonisten des zentralen Cannabinoid-Rezeptors CB1**
Use of central cannbinoid receptor CB1 agonists
Utilisation d'agonistes du récepteur central de cannabinoides BB1

(30) Priorität: 21.02.1997 DE 19706903
(43) Veröffentlichungstag der Anmeldung: 26.08.1998
(73) Patentinhaber: Bayer HealthCare AG, 51368 Leverkusen (DE)
(72) Erfinder: Mittendorf, Joachim, Dr., 42113 Wuppertal (DE); Dressel, Jürgen, Dr., 42477 Radevormwald (DE); Matzke, Michael, Dr., 42113 Wuppertal (DE); Franz, Jürgen, Dr., 42781 Haan (DE); Spreyer, Peter, Dr., 40225 Düsseldorf (DE); Vöhringer, Verena, Dr., 42113 Wuppertal (DE); Schuhmacher, Joachim, Dr., 42113 Wuppertal (DE); Friedl, Arno, Dr., 51427 Bergisch Gladbach (DE); Horvath, Ervin, Dr., 51373 Leverkusen (DE); Mauler, Frank, Dr., 51491 Overath (DE); De Vry, Jean-Marie-Viktor, Dr., 51503 Rösrath (DE); Jork, Reinhard, Prof. Dr., 51491 Overath (DE)

(56) Entgegenhaltungen:
- EP-A- 0 427 518
- WO-A-93/05031
- WO-A-94/12466
- WO-A-95/20958
- WO-A-95/33429
- WO-A-96/18391
- DE-C- 2 416 491
- US-A- 5 112 820
- SKAPER ET AL: "The ALIAmide palmitoylethanolamide and cannbinoids, but not anandamide are protective in a delayed postglutamate paradigm of excititoxic death in cerabellar granule neurons." PROC NATL ACAD SCI USA, Bd. 93, 1996, Seiten 3984-3989, XP002224112 USA
- SHOMAMI ET AL: "Long-tern effect of HU-211" BRAIN RES., Bd. 674, 1995, Seiten 55-62, XP002224113
- CONSROE: "Brain Cannbinoid Systems as Targets for Therapy of Neurological Disorders." NEUROBIOLOGY OF DISEASE, Bd. 5, 1998, Seiten 534--551, XP000920684

## Beschreibung

Die vorliegende Erfindung betrifft die Verwendung von bekannten Agonisten des zentralen Cannabinoid-Rezeptors CB1 zur Prophylaxe und Behandlung von neurodegenerativen Erkrankungen, insbesondere zur Behandlung von Apoplexia cerebri und Schädel-Hirn-Trauma.

Δ⁹-Tetrahydrocannabinol (Δ⁹-THC) und in geringem Maße auch Δ⁸-THC sind die biologisch aktiven Bestandteile in Extrakten der Pflanze Cannabis sativa (Marihuana, Haschisch) und sind verantwortlich für die Effekte auf das menschliche Zentrale Nervensystem (ZNS). Potentielle historische und kontemporäre therapeutische Anwendungen von Cannabis-Präparaten umfassen u.a. Analgesie, Emesis, Anorexie, Glaukom und Bewegungsstörungen.

Bislang wurden zwei Subtypen von Cannabinoid-Rezeptoren und eine Spleiß-Variante identifiziert. Der CB1-Rezeptor (Nature 1990, 346, 561) und eine Spleiß-Variante CB1a (J. Biol. Chem. 1995, 270, 3726) sind überwiegend im Zentralen Nervensystem lokalisiert. Der CB2-Rezeptor wurde überwiegend im peripheren Gewebe, insbesondere in Leukozyten, Milz und Makrophagen gefunden (Eur. J. Biochem. 1995, 232, 54).
CB1 und CB2-Rezeptoren besitzen sieben Transmembranregionen und gehören zur Familie der G-Protein-Rezeptoren. Beide Rezeptoren sind negativ gekoppelt via Gᵢ/Gₒ-Protein zur Adenylatcyclase und möglicherweise negativ gekoppelt zur präsynaptischen Freisetzung von Glutamat [vgl. J. Neurosci. 1996, 16, 4322]. CB1-Rezeptoren sind darüberhinaus positiv gekoppelt mit Kalium-Kanälen sowie negativ gekoppelt mit N- und Q-Typ Calcium-Kanälen.

Darüberhinaus ist bekannt, daß sich die Cannabinoid CB1-Rezeptor-Agonisten in 4 Klassen einteilen, die klassischen und nicht-klassischen Cannabinoide, die Aminoalkylindole und die Eicosanoide [vgl. Pharmacol. Rev. 1986, 38, 75; Eur. Med. Chem. 1996, 3, 101; Cannabinoid Receptors, R. Pertwee (Ed.), Academic Press, San Diego, 1995; J. Med. Chem. 1976, 19, 445; J. Med. Chem. 1976, 19, 454; J. Med. Chem. 1976, 19, 461; WO 95/33429; DE 2416491; J. Med. Chem. 1996, 36, 3875; US 4371720; Curr. Med. Chem. 1996, 3, 101; Curr. Pharm. Design 1995, 1, 343; Tetrahedr. Lett. 1994, 50, 2671; Life Sci. 1995, 56, 2007; Johnson, M.R., Melvin, L.S. In "Cannabinoids as Therapeutic Agents; Mechoulam R., Ed.; CRC Press, Boca Raton Fl 1986, pp. 121-145; J. Med. Chem. 1984, 7, 67; Pharmacol. Rev. 1986, 38, 1; Exp. Opin. Invest. Drugs 1996, 5, 1245; Pharmacol. Rev. 1986, 38, 151; Drug Design and Discovery 1995, 13, 155; J. Pharm. Exp. Ther. 1993, 265, 218; US 4391827; J. Med. Chem. 1995, 38, 3094; Bioorg. Med. Chem. Lett. 6, 1996, 17; J. Med. Chem. 1992, 35, 124; J. Med. Chem. 1991, 34, 1099; Bioorg. Med. Chem. Lett. 4, 1994, 563; Bioorg. Med. Chem. Lett. 5, 1995, 381; US 5112820; Tetrahedr. Lett. 1995, 1401; J. Med. Chem. 1996, 39, 4515; Drugs of Today 32, 1996, 275; J. Med. Chem. 1996, 39, 471; J. Med. Chem. 1994, 37, 1889; Mol. Pharmacol. 46, 516, 1994; Biochem. Pharmacol. 1995, 50, 83; J. Med. Chem. 1993, 36, 3032; Biochem. Pharmacol. 1994, 48, 1537; J. Biol. Chem. 1994, 269, 22937; Proc. Natl. Acad. Sci 1993, 90, 7656; J. Prostagl. Leukotr. Essen. Fatty Acids 1995, 52, 83; Science 1992, 258, 1946; Life Sci 1995, 56, 2041; FEBS Lett. 1994, 350, 240; Showalter V.M.; J. Pharmacol. Exp. Therap.1996, 989; Pharm. Res. 13, 1996, 62; J. Med. Chem. 1997, 40, 659].

Außerdem ist bekannt, daß Apoplexia cerebri eine Folge einer plötzlichen Durchblutungsstörung eines menschlichen Gehirnbereichs mit nachfolgenden Funktionsausfällen, mit entsprechenden neurologischen und/oder psychischen Symptomen ist. Die Ursachen für Apoplexia cerebri können in Hirnblutungen (z.B nach einem Gefäßriß bei Hypertonie, Arteriosklerose und apoplektischem Aneurysma) und Ischämien (z.B. durch eine Blutdruckabfallkrise oder Embolie) liegen. Die Funktionsausfälle im Gehirn führen zu einer Degeneration oder Abtötung der Gehirnzellen (vgl. Journal of Cerebral Blood Flow and Metabolism 1981, 1, 155; Chem. Eng. News 1996 (May 13), 41; Trends Pharmacol. Sci. 1996, 17, 227). Unter Schädel/Hirn-Trauma versteht man gedeckte und offene Schädelverletzungen mit Gehirnbeteiligung [vgl. Schweiz. med. Wschr. 1993, 123, 449].

Nach einen cerebralen Gefäßverschluß wird nur ein Teil des Gewebevolumens als direkte Folge der Minderdurchblutung und der damit verbundenen verminderten Sauerstoffversorgung zerstört [vgl. Neurology 1996, 47, 884]. Dieser als lnfarktkern bezeichnete Gewebebereich läßt sich nur durch sofortige Rekanalisation des Gefäßverschlusses, z.B. durch lokale Thrombolyse, vor dem Absterben bewahren und wird deshalb nur bedingt einer Therapie zugänglich sein. Die äußere vom Volumen her mindestens ebenso große Randzone, die auch als Penumbra bezeichnet wird, stellt zwar ebenfalls unmittelbar nach Eintritt des Gefäßverschlusses ihre Funktion ein, wird durch die Kollateralversorgung aber zunächst noch ausreichend mit Sauerstoff versorgt und erst nach einigen Stunden bzw. sogar erst nach Tagen, irreversibel geschädigt. Da der Zelltod in diesem Gebiet nicht sofort eintritt, eröffnet sich eine therapeutische Chance, die für den Krankheitsverlauf sowohl nach Schlaganfall als auch nach Trauma ungünstige Entwicklung zu blockieren.

Die zahlreichen therapeutischen Ansätze zur Reduktion des Infarktvolumens um- , fassen beispielsweise die Blockierung von Glutamat-Rezeptoren bzw. der Glutamat-Freisetzung, Radikal-Fänger, entzündungshemmende Substanzen, Substanzen zur Blockierung spannungsabhängiger Calcium- bzw. Natriumkanäle, GABA-Agonisten [vgl. Trends Pharmacol. Sci. 17, 1996, 227].

Die Hemmung der glutamatergen Neurotransmission bzw. Hemmung der Glutamat-Freisetzung kann durch eine Vielzahl von unterschiedlich pharmakologisch wirkenden Substanzen und damit unterschiedlichen Wirkmechanismen erreicht werden [GABA Rezeptor Liganden (Neurosci. Lett 1990, 118, 99, Br. J. Pharmacol. 1997, 120, 60), Aluminium (Neurotoxicol. 1992, 13, 413), Ethanol (Eur. J. Pharmacol. 1992, 219, 469), Barbiturate, beispielsweise Thiopental (Br. J. Pharmacol. 1996, 119, 1498), Adenosin A1-Rezeptoren (Neurosci. Lett. 1996, 220, 163), α₂-Agonisten (Anesthesiol. 1996, 85, 551), Cannabinoid Rezeptor Agonisten (J. Neurosci. 1996, 16 4322).

Für Kynurenic Acid (Brain Res. 1992, 592, 333) und Theophyllin (Brain Res 1991, 565, 353) wurde gezeigt, daß diese Substanzen, obwohl sie die Glutamat-Freisetzung in Vitro deutlich hemmen, in vivo keine neuroprotektive Wirkung haben.

Im Gegensatz zur Spekulation von Shen et al. (J. Neurosci. 1996, 16, 4322) ist der Cannabinoid Rezeptor Agonist HU210, das (-)-Enantiomer des am Cannabinoid Rezeptor inaktiven HU-211, in einem Schädel-Hirntraumamodell nicht neuroprotektiv (J. Neurotrauma 1993, 10, 109).

Es wurde nun gefunden, daß die oben zitierten bekannten Cannabinoid CB1-Rezeptor-Agonisten überraschenderweise geeignet sind zur Prophylaxe und Behandlung von neurodegenerativen Erkrankungen, insbesondere von Apoplexia cerebri und Schädel/Hirn-Trauma.

Bevorzugt verwendet werden [A] bekannte Agonisten des zentralen Cannabinoid-Rezeptors CB1 der allgemeinen Formel (I) 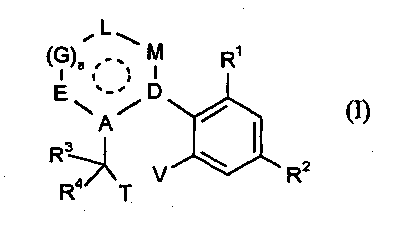 in welcher
- A und D: gleich oder verschieden sind und in Abhängigkeit von der Lage einer Einfach- oder Doppelbindung für ein C-Atom oder für die CH-Gruppe stehen, .
- E: in Abhängigkeit von der Lage einer Einfach- oder Doppelbindung für die CH- oder CH₂-Gruppe oder für ein Schwefelatom steht,
- G, L und M: gleich oder verschieden sind und in Abhängigkeit von der Lage einer Einfach- oder Doppelbindung für einen Rest der Formel -CR⁵, -CR⁶R⁷ oder N-R⁸ stehen,
worin
R⁵, R⁶, R⁷ und R⁸ gleich oder verschieden sind und Wasserstoff, Hydroxy, Formyl, (C₂-C₆)-Alkenyl, (C₂-C₆)-Alkinyl bedeuten, oder
(C₁-C₆)-Alkyl bedeuten, das gegebenenfalls durch Hydroxy oder (C₁-C₄)-Alkoxy substituiert ist,
oder
R⁶ und R⁷ gemeinsam für einen Rest der Formel =O stehen,
- a: für ein Zahl 0 oder 1 steht,
- R¹: für Wasserstoff oder Hydroxy steht, oder für (C₁-C₁₁)-Alkyl, (C₁-C₆)-Alkoxy, (C₁-C₆)-Alkoxycarbonyl oder (C₁-C₄)-Acyloxy steht, die gegebenenfalls durch Hydroxy, (C₁-C₁₀)-Alkoxy oder durch eine Gruppe der Formel -NR⁹R¹⁰ substituiert sind,
worin
R⁹ und R¹⁰ gleich oder verschieden sind und Wasserstoff, Phenyl, (C₁-C₄)-Alkyl bedeuten,
R⁹ und R¹⁰ gemeinsam mit dem Stickstoffatom einen 5- bis 7-gliedrigen gesättigten Heterocyclus bilden, der gegebenenfalls noch ein weiteres Heteroatom aus der Reihe S und O oder einen Rest der Formel -NR¹¹ enthalten kann, oder
R¹¹ Wasserstoff, Phenyl, (C₁-C₆)-Alkyl oder (C₁-C₆)-Acyl bedeutet,
worin
- R²: für (C₁-C₁₀)-Alkyl oder (C₁-C₁₀)-Alkoxy steht, die gegebenenfalls durch Phenyl, Halogen, Hydroxy, Azido, Nitro, Trifluormethyl, Trifluormethoxy, Carboxyl, (C₁-C₄)-Alkoxy oder (C₁-C₄)-Alkoxycarbonyl oder durch eine Gruppe der Formel -NR¹²R¹³ substituiert sind,
worin
R ¹² und R¹³ gleich oder verschieden sind und die oben angegebene Bedeutung von R⁹ und R¹⁰ haben,
- R³ und R⁴: gleich oder verschieden sind und für Wasserstoff oder für (C₁-C₆)-Alkyl stehen, das gegebenenfalls durch Hydroxy substituiert ist,
oder
- R³ und R⁴: gemeinsam für einen Rest der Formel H₂C= stehen,
- T: für (C₁-C₆)-Alkyl oder (C₂-C₆)-Alkenyl steht,
und
- V: für Hydroxy steht,
oder
- T und V: gemeinsam unter einem Ringschluß, für ein Sauerstoffatom oder für einen Rest der Formel -NR¹⁴ stehen,
worin
R¹⁴ Wasserstoff oder Methyl bedeutet
mit Ausnahme von Verbindungen folgender Konfiguration 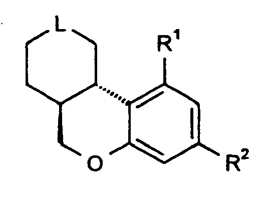 [B] nicht-klassischer Cannabinoide der allgemeinen Formel (Ia) 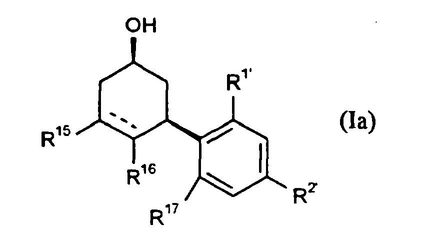 in welcher
- R^{1'} und R^{2'}: gleich oder verschieden sind und die oben angegebene Bedeutung von R¹ und R² haben,
- R¹⁵ und R¹⁶: gleich oder verschieden sind und für Wasserstoff oder (C₁-C₈)-Alkyl stehen, das gegebenenfalls durch Hydroxy substituiert ist,
oder
- R¹⁵ und R¹⁶: gemeinsam unter Einbezug der C-C-Bindung einen Phenylring oder einen 3- bis 7-gliedrigen carbocyclischen Ring bilden, wobei die Ringsysteme gegebenenfalls durch (C₁-C₆)-Alkoxycarbonyl oder durch (C₁-C₈)-Alkyl substituiert sind, das seinerseits durch Hydroxy substituiert sein kann,
- R¹⁷: für Wasserstoff steht,
oder
R¹⁶ und R¹⁷ gemeinsam einen 6-gliedrigen gesättigten, partiell gesättigten oder ungesättigten Heterocyclus bilden, der ein Heteroatom aus der Reihe S und O oder einen Rest der Formel -NR¹⁸ enthalten kann,
worin
R¹⁸ Wasserstoff oder (C₁-C₄)-Alkyl bedeutet,
und wobei die Ringsysteme gegebenenfalls bis zu 3-fach gleich oder verschieden, auch geminal, durch (C₁-C₈)-Alkyl substituiert sind, das seinerseits durch Hydroxy substituiert sein kann, [C] der Aminoalkylindole der allgemeinen Formeln (Ib) und (Ic) 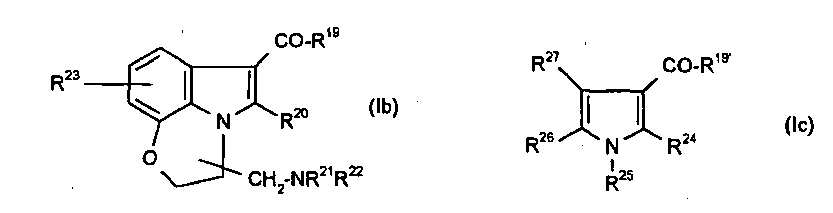 in welcher
- R¹⁹ und R^{19'}: gleich oder verschieden sind und für (C₆-C₁₀)-Aryl oder für einen 5- bis 7-gliedrigen, gesättigten oder ungesättigten Heterocyclus mit bis zu 3 Heteroatomen aus der Reihe S, N und/oder O stehen, die gegebenenfalls durch einen oder mehreren, gleichen oder verschiedenen Substituenten substituiert sind, die aus der Gruppe ausgewählt sind, die besteht aus: Nitro, Halogen, Trifluormethyl, Hydroxy, Carboxyl oder durch (C₁-C₅)-Acyl, (C₁-C₆)-Alkoxy, (C₁-C₆)-Alkylthio, (C₁-C₆)-Alkoxycarbonyl und (C₁-C₆)-Alkyl, das seinerseits durch Hydroxy substituiert sein kann,
- R²⁰: für Wasserstoff oder Methyl steht,
- R²¹ und R²²: gleich oder verschieden sind und für Wasserstoff oder für (C₁-C₆)-Alkyl stehen, oder
- R²¹ und R²²: gemeinsam mit dem Stickstoffatom einen 5- bis 7-gliedrigen, gesättigten oder partiell gesättigten Heterocyclus bilden, der gegebenenfalls ein weiteres Sauerstoff- oder Schwefelatom oder einen Rest der Formel -NR²⁸ enthalten kann,
worin
R²⁸ die oben angegebene Bedeutung von R⁸ hat und mit dieser gleich oder verschieden ist,
- R²³: für Wasserstoff, Halogen, Hydroxy, (C₁-C₈)-Alkyl oder für (C₁-C₈)-Alkoxy steht,
- R²⁴: für Wasserstoff oder für (C₁-C₆)-Alkyl steht,
- R²⁵: für Wasserstoff, Phenyl, Cycloalkyl mit 3 bis 8 Kohlenstoffatomen, oder für (C₁-C₆)-Alkyl steht, das gegebenenfalls durch eine Gruppe der Formel -NR²⁹R³⁰ substituiert ist,
worin
R²⁹ und R³⁰ gleich oder verschieden sind und die oben angegebene Bedeutung von R⁹ und R¹⁰ haben ,
R²⁶ und R²⁷ für Wasserstoff stehen, oder gemeinsam unter Einbezug der Doppelbindung einen Phenylring bilden,
[D] Verbindungen der allgemeinen Formel (Id) 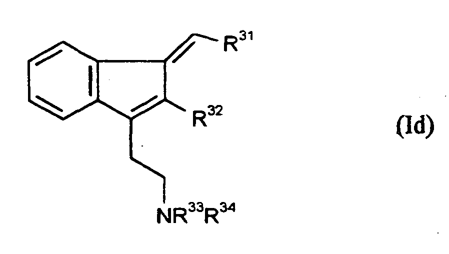 in welcher
- R³¹: die oben angegebene Bedeutung von R¹⁹ hat und mit dieser gleich oder verschieden ist,
- R³²: für Wasserstoff, (C₁-C₃)-Alkyl oder für (C₁-C₃)-Alkoxy steht,
- R³³ und R³⁴: die oben angegebene Bedeutung von R²¹ und R²² haben und mit dieser gleich oder verschieden sind,
und [E] Eicosanoide der allgemeinen Formel (Ie) 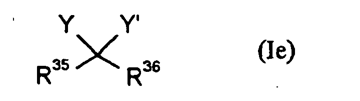 in welcher
- Y und Y': für Wasserstoff stehen,
oder
- Y und Y': gemeinsam für einen Rest der Formel =O oder =S stehen,
- R³⁵: für (C₁₆-C₃₀)-Alkenyl mit mindestens drei Doppelbindungen steht,
- R³⁶: für Trifluormethyl oder für eine Gruppe der Formel -OR³⁷ oder -NR³⁸R³⁹ steht,
worin
R³⁷ Wasserstoff oder (C₁-C₁₀)-Alkyl bedeutet, das gegebenenfalls mit einem oder mehreren, gleichen oder verschiedenen Substituenten substituiert ist, die aus der Gruppe ausgewählt sind, die besteht aus: Hydroxy, Halogen, Trifluormethyl, (C₆-C₁₀)-Aryl und (C₁-C₆)-Alkoxy,
R³⁸ und R³⁹ die oben angegebene Bedeutung von R³⁷ haben und mit dieser gleich oder verschieden sind,
oder
R³⁸ und R³⁹ gemeinsam mit dem Stickstoffatom einen 5- bis 7-gliedrigen gesättigten Heterocyclus bilden, der gegebenenfalls ein weiteres Heteroatom aus der Reihe S und O oder eine Gruppe der Formel -NR⁴⁰ enthalten kann,
worin
R⁴⁰ die oben angegebene Bedeutung von R⁸ hat und mit dieser gleich oder verschieden ist,
und deren Salze und isomere Formen,
bei der Bekämpfung von neurodegenerativen Erkrankungen, insbesondere Apoplexia Cerebri und Schädel/Hirn-Trauma.

Die erfindungsgemäß verwendeten Verbindungen können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure. Ganz besonders bevorzugt sind die oben aufgeführten Salze, die sich durch die Aminfunktion bilden.

Physiologisch unbedenkliche Salze können ebenso Metall- oder Ammoniumsalze der erfindungsgemäßen Verbindungen sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di- bzw. Triethylamin, Di- bzw. Triethanolamin, Dicyclohexylamin, Dimethylaminoethanol, Arginin, Lysin, Ethylendiamin oder 2-Phenylethylamin.

Im Rahmen der vorliegenden Erfindung haben die Substituenten im allgemeinen die folgende Bedeutung:

(C₁-C₁₁)- (C₁-C₁₀)- (C₁-C₈)-, (C₁-C₆)-, (C₁-C₄)- und (C₁-C₃)-Alkyl stehen in Abhängigkeit von den oben aufgeführten Substituenten im allgemeinen für einen geradkettigen oder verzweigten Kohlenwassserstoffrest mit 1 bis 11 Kohlenstoffatomen. Beispielsweise seien Methyl, Ethyl, Propyl, Isopropyl, Butyl, Isobutyl, Pentyl, Isopentyl, Hexyl, Isohexyl, Heptyl, Isoheptyl, Octyl und Isooctyl genannt.

(C₁₆-C₃₀)- und (C₂-C₆)- Alkenyl stehen in Abhängigkeit von den oben aufgeführten Substituenten im allgemeinen für einen geradkettigen oder verzweigten Kohlenwasserstoffrest mit 2 bis 30 Kohlenstoffatomen und einer oder mehreren, bevorzugt mit einer, zwei bzw. mindestens 3 Doppelbindungen. Beispielsweise seien Allyl, Propenyl, Isopropenyl, Butenyl, Isobutenyl, Pentenyl, Isopentenyl, Hexenyl, Isohexenyl, Heptenyl, Isoheptenyl, Octenyl und Isooctenyl genannt. (C₂-C₆)-Alkinyl steht im allgemeinen für einen geradkettigen oder verzweigten Kohlenwasserstoffrest mit 2 bis 6 Kohlenstoffatomen und einer oder mehreren, bevorzugt mit einer oder zwei Dreifachbindungen. Bevorzugt ist der Niederalkylrest mit 2 bis etwa 5 Kohlenstoffatomen und einer Dreifachbindung. Besonders bevorzugt ist ein Alkenylrest mit 2 bis 4 Kohlenstoffatomen und einer Dreifachbindung. Beispielsweise seien Acetylen, 2-Butin, 2-Pentin und 2-Hexin genannt.

Cycloalkyl steht im allgemeinen für einen cyclischen Kohlenwasserstoffrest mit 3 bis 8 Kohlenstoffatomen. Bevorzugt ist der Cyclopropan-, Cyclopentan- und der Cyclohexanring. Beispielsweise seien Cyclopentyl, Cyclohexyl, Cycloheptyl und Cyclooctyl genannt.

Aryl steht im allgemeinen für einen aromatischen Rest mit 6 bis 10 Kohlenstoffatomen. Bevorzugte Arylreste sind Phenyl und Naphthyl.

(C₁-C₁₀)-, (C₁-C₆)- und (C₁-C₄)-Alkoxy stehen in Abhängigkeit von den oben aufgeführten Substituenten im allgemeinen für einen über ein Sauerstoffatom gebundenen geradkettigen oder verzweigten Kohlenwasserstoffrest mit 1 bis 10 Kohlenstoffatomen. Beispielsweise seien Methoxy, Ethoxy, Propoxy, Isopropoxy, Butoxy, Isobutoxy, Pentoxy, Isopentoxy, Hexoxy, Isohexoxy, Heptoxy, Isoheptoxy, Octoxy oder Isooctoxy genannt.

(C₁-C₆)- und (C₁-C₅)-Acyl stehen im allgemeinen für geradkettiges oder verzweigtes Niedrigalkyl mit 1 bis 6 Kohlenstoffatomen, die über eine Carbonylgruppe gebunden sind. Beispielsweise seien genannt: Acetyl, Ethylcarbonyl, Propylcarbonyl, Isopropylcarbonyl, Butylcarbonyl und Isobutylcarbonyl.

(C₁-C₆)- und (C₁-C₄)-Alkoxycarbonyl können beispielsweise durch die Formel 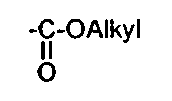 dargestellt werden.
Alkyl steht hierbei für einen geradkettigen oder verzweigten Kohlenwasserstoffrest mit 1 bis 6 und 1 bis 4 Kohlenstoffatomen. Beispielsweise seien die folgenden Alkoxycarbonylreste genannt: Methoxycarbonyl, Ethoxycarbonyl, Propoxycarbonyl, Isopropoxycarbonyl, Butoxycarbonyl oder Isobutoxycarbonyl.

Halogen steht im Rahmen der Erfindung für Fluor, Chlor, Brom und Jod.

(C₁-C₆)-Alkylthio steht im allgemeinen für einen über ein Schwefelatom gebundenen geradkettigen oder verzweigten Kohlenwasserstoffrest mit 1 bis 6 Kohlenstoffatomen. Beispielsweise seien Methylthio, Ethylthio und Propylthio genannt.

(C₁-C₄)-Acyloxy kann beispielsweise durch die Formel 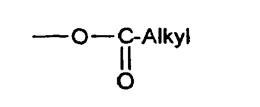 dargestellt werden.
Alkyl steht hierbei für einen geradkettigen oder verzweigten Kohlenwasserstoffrest mit 1 bis 4 Kohlenstoffatomen. Beispielsweise seien die folgenden Acyloxyreste genannt: Methylcarbonyloxy, Ethylcarbonyloxy und Propylcarbonyloxy.

Gesättigter, partiell gesättigter und ungesättigter Heterocyclus steht im Rahmen der Erfindung im allgemeinen für einen 5- bis 7-gliedrigen, vorzugsweise 5- bis 6-gliedrigen Heterocyclus der bis zu 3 Heteroatome aus der Reihe S, N und/oder O enthalten kann. Beispielsweise seien genannt: Pyridyl, Thienyl, Furyl, Pyrrolyl, Pyrrolidinyl, Piperazinyl, Pyrimidyl, Thiazolyl, Oxazolyl, Imidazolyl, Morpholinyl oder Piperidyl.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweilige Mischungen. Diese Mischungen der Enantiomeren und Diastereomeren lassen sich in bekannter Weise in die stereoisomer einheitlichen Bestandteile trennen.

Bevorzugt verwendet werden erfindungsgemäße Verbindungen der allgemeinen Formel (I),
in welchen in den Verbindungen der allgemeinen Formel (I)
A, D, E, G, a, L, M, T und V die oben angegebene Bedeutung haben,
- R¹: für Hydroxy oder für den Rest der Formel -O-CO-CH₃ oder -O-CO-(CH₃)-N(C₂H₅)₂ steht,
- R³ und R⁴: gemeinsam für den =CH₂-Rest stehen
oder
- R³ und R⁴: für Wasserstoff Methyl oder für den (CH₂)₃-OH-Rest stehen,
in welchen in den Verbindungen der allgemeinen Formel (Ia),
- R^{2'} und R¹⁷: die oben angegebene Bedeutung haben,
- R^{1'}: für Hydroxy steht,
- R¹⁵ und R¹⁶: für Wasserstoff stehen, oder gemeinsam unter Einbezug der C-C-Bindung einen Pyridyl- oder CH-OH-substituierten Phenylring bilden,
in welchen in den Verbindungen der allgemeinen Formel (Ib)
- R¹⁹: für Naphthyl steht,
- R²⁰: für Methyl steht,
- R²³: für Wasserstoff steht,
und
- R²¹ und R²²: gemeinsam mit dem Stickstoffatom einen Morpholinring bilden,
in welchen in den Verbindungen der allgemeinen Formel (Ic)
- R^{19'}: für Methoxy-substituiertes Naphthyl steht,
- R²⁴: für Wasserstoff steht,
- R²⁵: für Phenyl, (C₁-C₆)-Alkyl oder für den Rest 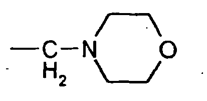 steht,
- R²⁶ und R²⁷: für Wasserstoff oder Phenyl stehen,
in welchen in den Verbindungen der allgemeinen Formel (Id)
- R³¹: für Naphthyl steht, das gegebenenfalls durch Methoxy substituiert ist,
- R³²: für Wasserstoff oder Methyl steht,
- R³³ und R³⁴: gemeinsam mit dem Stickstoffatom für Morpholin stehen,
in welchen in den Verbindungen der allgemeinen Formel (Ie)
- Y und Y': für Wasserstoff oder gemeinsam für den =O-Rest stehen,
- R³⁵: für (C₁₆-C₂₁)-Alkenyl steht,
und
- R³⁶: für Trifluormethyl oder für den Rest der Formel -NR³⁸R³⁹ steht,
worin.
- R³⁸ und R³⁹: gleich oder verschieden sind und Wasserstoff oder (C₁-C₃)-Alkyl bedeuten, das gegebenenfalls durch Fluor oder Hydroxy substituiert ist,
und deren Salze und isomere Formen,
bei der Bekämpfung von neurodegenerativen Erkrankungen, insbesondere Apoplexia Cerebri und Schädel/Hirn-Trauma.

Ganz besonders bevorzugt verwendet werden folgende Verbindungen: 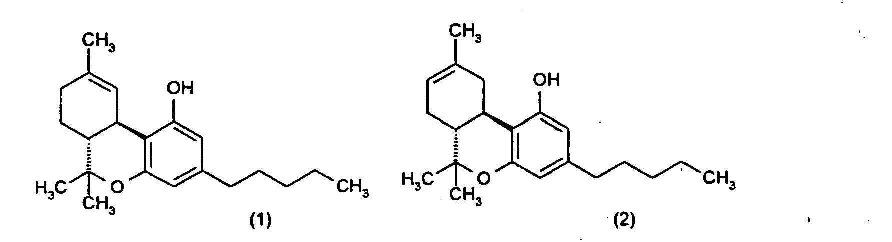 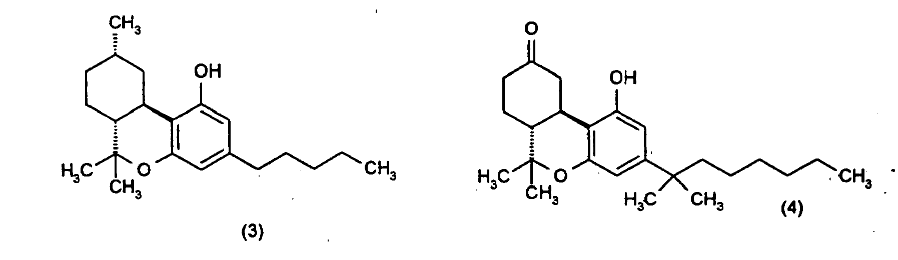 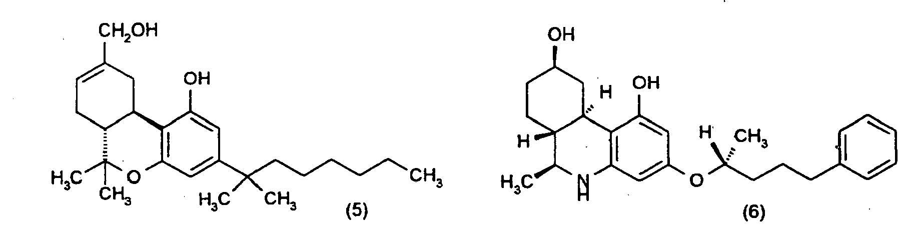 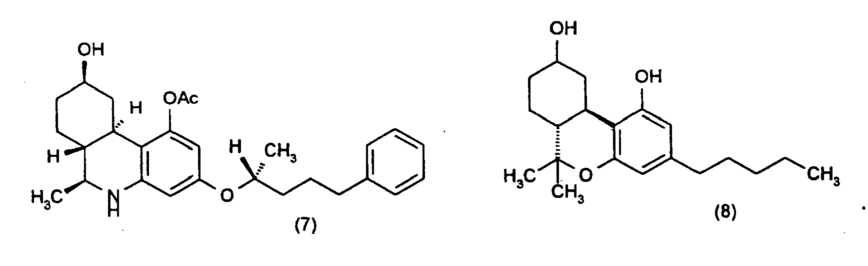 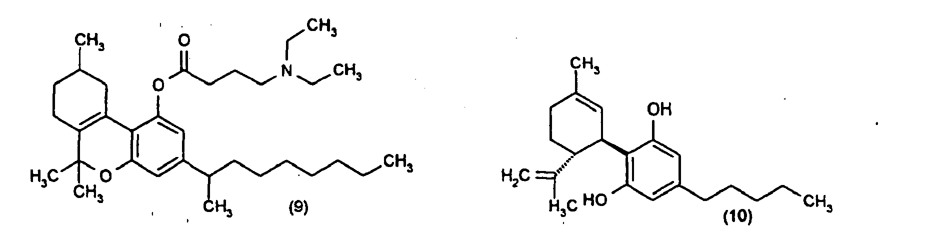 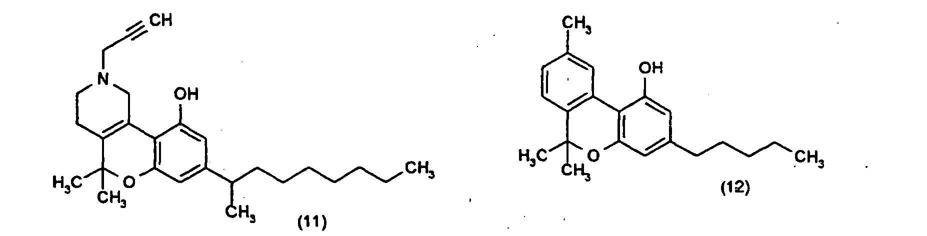 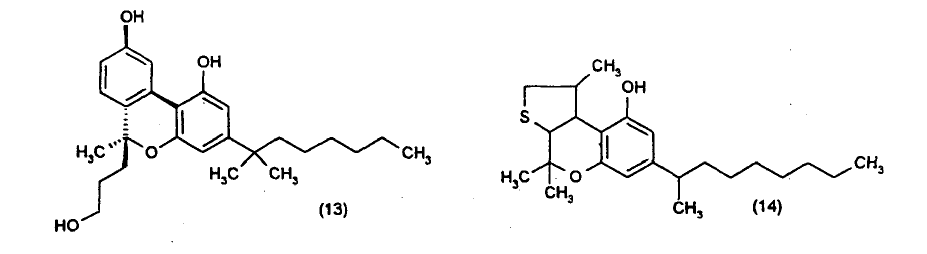 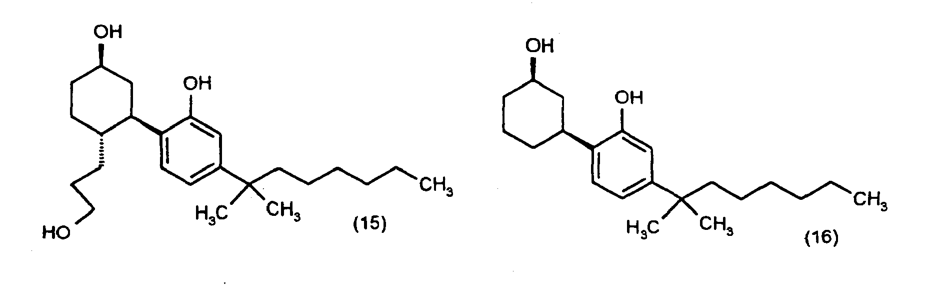 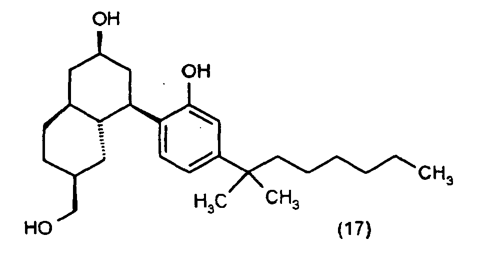 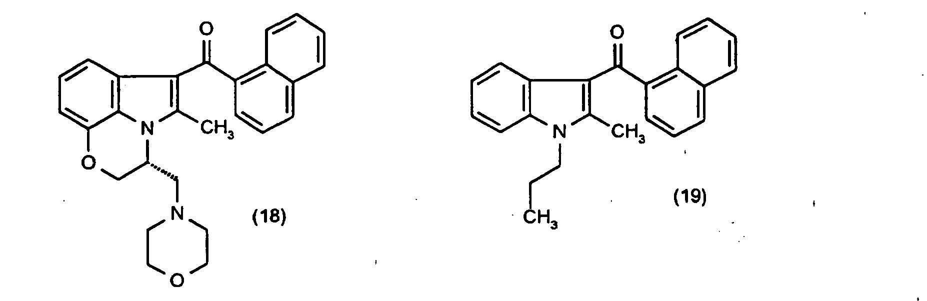 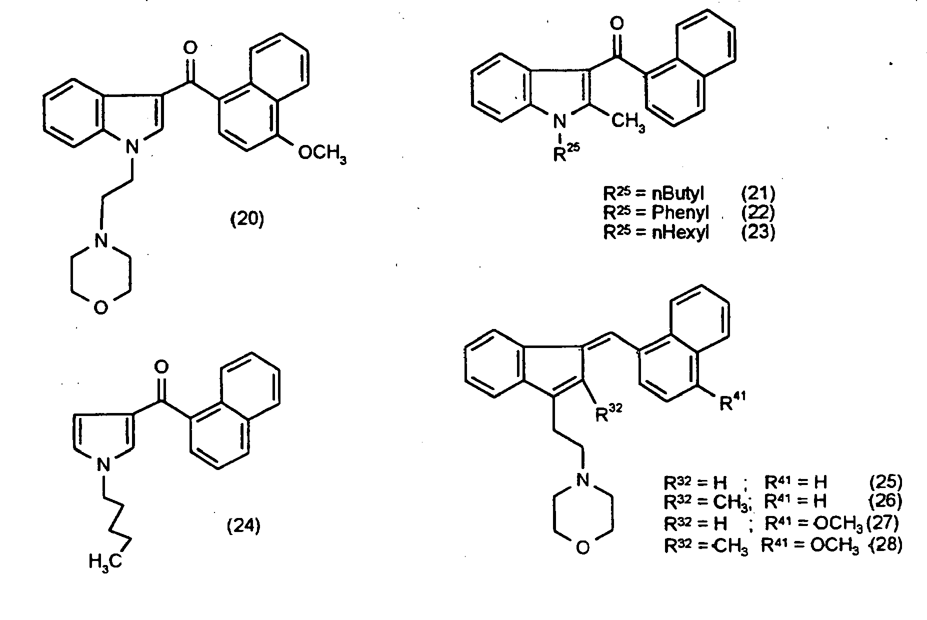 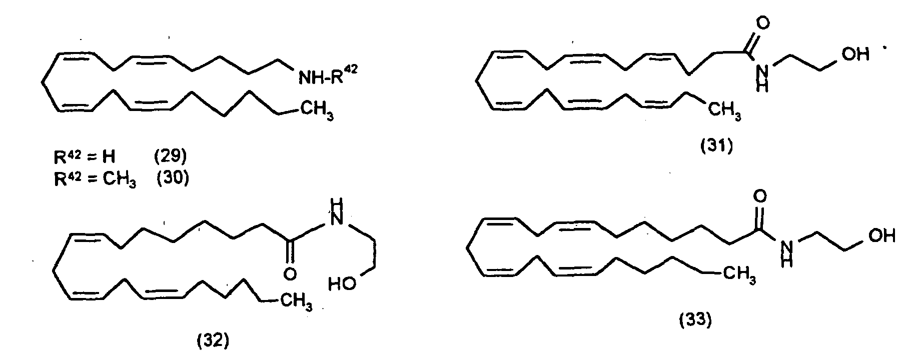 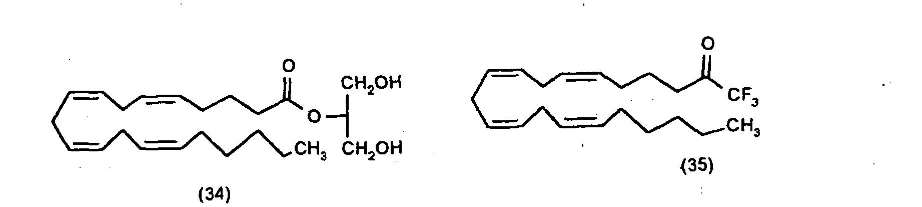 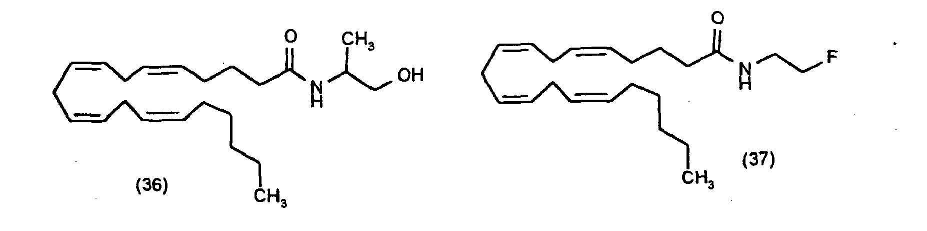 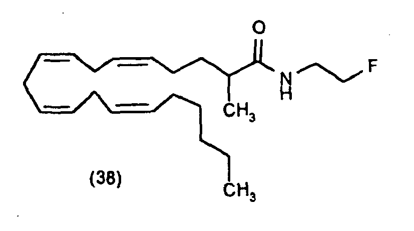 zur Prophylaxe und Behandlung von neurodegenerativen Erkrankungen, insbesondere zur Behandlung von Apoplexia cerebri und Schädel-Hirn-Trauma.

Die oben aufgeführten bekannten Verbindungen können nach üblichen Methoden hergestellt werden [vgl. die oben aufgeführen Literaturstellen].

Cannabinoid-Rezeptor CB-1 Agonisten im Sinne der Erfindung sind Verbindungen, die in dem nachfolgend beschriebenen CB-1 Luziferase Reporter-Gentest einen IC₅₀-Wert von weniger als 10⁻⁵ M aufweisen.

### CB1-Luciferase Reportergen Test

### 1. Klonierung des Ratten Cannabinoid Rezeptors CB1

Gesamt-RNA aus Ratten-Hirn (das Gewebe wurde frisch getöteten Tieren entnommen und in flüssigem Stickstoff schockgefroren) wurde durch saure Guanidinium-Thiocyanat/Phenol/Chloroform-Extraktion (J. Biol. Chem. 1979, 18, 5294) isoliert und mittels reverser Transkriptase und Random-Primern (jeweils von Invitrogen) in cDNA überführt. Die Polymerase Ketten Reaktion (PCR, Bedingungen: 4 min 94°C, 1x; 1 min 94°C; 2 min 53°C; 1 min 72°C, 50 Zyklen; 1 min 94°C, 2 min 53°C, 4 min 72°C, 1x) wurde in einem Perkin Elmer Thermocycler mit dem Enzym Taq Polymerase (Perkin Elmer) durchgeführt; die eingesetzten Oligonukleotid-Primer (Basen 99 bis 122: 5'→3', "down"; 1556-1575: 3'←5', "up") waren von der publizierten Sequenz des Ratten Cannabinoid- Rezeptors (Nature 1990, 346, 561) abgeleitet und wurden auf einem DNA Synthesizer, Modell 1380 der Fa. Applied Biosystems, synthetisiert. Ein Teil der PCR-Reaktion wurde in einem 1 %igen Agarose-Gel in 1x TBE-Puffer aufgetrennt und anschließend mit Ethidium-Bromid angefärbt, wobei nur eine Bande mit der erwarteten Länge sichtbar war (etwa 1,5 kb). Dieses PCR-Produkt wurde in den TA-Cloning Vektor (Invitrogen) subkloniert und die Nukleotid-Sequenz des Inserts mit T7DNA Polymerase (Sequenase, USA/Amersham) durch die Dideoxynukleotid-Kettenabbruch-Reaktion bestimmt. Das Insert besitzt eine Länge von 1477 Basenpaaren und enthält ein offenes Leseraster von 1419 Basenpaaren was einem Protein von 473 Aminosäuren entspricht. Die Anzahl der Basenpaare, die Position des offenen Leserasters und die Anzahl der Aminosäuren stimmen mit der publizierten Sequenz überein. Computer-Analysen wurden mit Hilfe der GCG Software Suite (Genetic Computer Group) durchgeführt. Das cDNA Insert wurde nach Partialverdauung mit HindIII und NotI (Biolabs) in den Expressionsvektor pRc/CMV (Invitrogen) subkloniert. Dieses Konstrukt (Plasmid CMV-RH) wurde für Transfektions-Experimente eingesetzt.

### 2. Stabile Transfektion der CHOluc9 Reporter Zellen

CHOluc9 Zellen wurden in 50 % Dulbecco's modifiziertem Eagle Medium / 50 % F-12 (DMEM/F12) gezüchtet, das 10 % foetales Kälberserum (FCS) enthielt. Transfektionen wurden in 6-well Platten angesetzt. 7,5 µg Qiagen-gereinigte CMV-RH Plasmid DNA wurden pro 10⁵ Zellen mit dem DOTAP Transfektions System zugegeben, entsprechend dem Versuchsprotokoll des Herstellers (Boehringer Mannheim). Transfizierte Zellen wurden mit 1 mg/ml G418 selektioniert und Einzelklone wurden durch Limiting Dilution auf 96-well Platten erhalten. Zellinien, die den Cannabinoid-Rezeptor exprimieren, wurden nach Inkubation mit dem Cannabinoid-Rezeptor Agonisten, WIN-55,212-2, in Gegenwart von Forskolin an der Hemmung der Reportergen-Expression identifiziert. Mehrere stabil transfizierte und subklonierte Zellinien wurden mittels RT-PCR, wie unter 1. beschrieben, weiter charakterisiert.

### 3. Test-Optimierung und pharmakologische Charakterisierung der CHOCB1 Reporter-Zellinie

Der Luciferase-Test wurde mit dem Ziel hoher Sensitivität und Reproduzierbarkeit, geringer Varianz und guter Eignung für die Durchführung auf dem Robotersystem optimiert durch Variation mehrerer Testparameter, wie z.B. Zelldichte, Dauer der Anzuchtphase und der Testinkubation, Forskolin-Konzentration, Medium-Zusammensetzung. Zur pharmakologischen Charakterisierung der Zellen und zum Roboter-gestützten Substanz-Screening wurde das folgende Testprotokoll verwendet: Die Stammkulturen wurden in 50 % Dulbecco's modifiziertem Eagle Medium / 50 % F-12 (DMEM/F12) mit 10 % FCS bei 37°C unter 10 % CO₂ gezüchtet und jeweils nach 2 bis 3 Tagen 1:10 gesplittet. Testkulturen wurden mit 5000 Zellen pro Napf in 96-well Platten ausgesät und 70 Stunden bei 37°C angezogen. Dann wurden die Kulturen vorsichtig mit Phosphat-gepufferter Saline gewaschen und mit serumfreiem Ultra-CHO Medium (Bio-Whittaker) rekonstituiert. Die in DMSO gelösten Substanzen wurden 1 x in Medium verdünnt und zu den Testkulturen pipettiert (maximale DMSO-Endkonzentration im Testansatz: 0,5 %). 20 Minuten später wurde Forskolin zugegeben und die Kulturen anschließend 3 Stunden im Brutschrank bei 37°C inkubiert. Danach wurden die Überstände entfernt und die Zellen durch Zugabe von 25 µl Lysereagens (25 mM Triphosphat, pH 7,8 mit 2mM DTT, 10 % Glycerin, 3 % TritonX100) lysiert.

Direkt danach wurde Luciferase Substrat Lösung (2,5mM ATP, 0,5 mM Luciferin, 0,1 mM Coenzym A, 10mM Tricin, 1,35mM MgSO₄, 15mM DTT, pH 7,8) zugegeben, kurz geschüttelt, und die Luciferase-Aktivität mit einem Hamamatzu Kamerasystem gemessen.

Zur Inaktivierung von Gᵢ-Proteinen wurden die Testkulturen vor dem Test für 16 Stunden mit 5 ng/ml (Endkonz.) Pertussis Toxin behandelt.

Die IC50-Werte wurden mit dem Programm GraphPadPrism berechnet (Hill-Gleichung, speziell: one-site competition).

| | **CBI-Luciferase Reportergen Test** |
|---|---|
| Beispiel | IC₅₀ [nM] |
| 1 | 13,32 |
| 5 | 0,48 |
| 15 | 0,23 |
| 18 | 1,55 |

### Bindungsstudien an Ratten Cortex Membranen

Membranprotein wird nach Standardmethoden aus unterschiedlichen Geweben bzw. von Zellen präpariert. Puffer, markierter Ligand, DMSO oder Teststubstanz werden zusammenpipettiert, anschließend werden 100 µg Protein hinzugegeben, die Mischung gut vermischt und 60 min bei 30°C im Wasserbad inkubiert. Nach Ablauf der Inkubationszeit wird die Reaktion durch Zugabe von eiskaltem Inkubationspuffer in jedes Röhrchen gestoppt. Nach Abfiltrieren wird mit 3/4 ml Inkubationspuffer nachgewaschen. Die Filter werden in Minivials überführt, die Radioaktivität wird in einem Flüssigszintillationszähler bestimmt.

| | **Bindung an Ratten Cortex Membranen** |
|---|---|
| Beispiel | Kᵢ [nM] |
| 1 | 1,69 |
| 5 | 1,00 |
| 15 | n.t. |
| 18 | 0,06 |

### Inhibition der Glutamat-Freisetzung

Nach Dekapitieren einer Ratte wird der Schädel eröffnet, das Gehirn herausgehoben und entlang der Mittelfurche durchschnitten. Der Hippocampus wird freipräpariert, vom restlichen Gewebe getrennt, in 350 µM dicke Schnitte geschnitten und für 60 min in Siebgefäßen bei 37°C inkubiert. Gefolgt von Basalwert und Stimulation 1 mit 75 mM KCI (S1) werden die Schnitte mit Testsubstanz inkubiert und dann die Stimulation mit KCl und Testsubstanz (S2) wiederholt. Die Glutamat-Konzentration der zu untersuchenden Proben wird dann über eine enzymatische Reaktion (GLDH) und fluorometrischer Messung von NADH gemessen. Anhand einer Eichkurve wird der Glutamatgehalt der Probe bestimmt, und unter Kenntnis des Proteingehaltes kann der Glutamatgehalt/mg Protein errechnet werden. Verglichen wird das Verhältnis S2/S1, Glutamat-Freisetzungsinhibitoren reduzieren dieses Verhältnis konzentrationsabhängig.

| | **Inhibition der Glutamat-Freisetzung** |
|---|---|
| Beispiel Nr. | % Inhibition @ 1 µM |
| 1 | no effect |
| 5 | 20 |
| 15 | |
| 18 | 50 |

### Hypothermie

### 1. Agonismus Prüfung:

Fünf Minuten nach Bestimmung der Basal-Körpertemperatur via Oesophagus Temperatursonde wird die Prüfsubstanz (i.v.) appliziert. Eine Kontrollgruppe erhält, ebenfalls i.v., nur das Lösungsmittel der Prüfsubstanzen. Die Körpertemperatur wird 7,5, 15, 30 und 60 Minuten nach i.v.-Applikation gemessen. Die Gruppengröße pro Dosis beträgt 5-7 Tiere (Ratten).

| | **Hypothermie** |
|---|---|
| Beispiel-Nr. | ED_{-1°C}^{a)} [mg/kg i.v.] |
| 1 | 0,8 |
| 5 | 0,01 |
| 15 | 0,03 |
| 18 | 0,2 |

| | |
|---|---|
| ^{a)} notwendige Dosis, um eine maximale Temperatursenkung von 1°C zu erreichen | |

### 2.Antagonismus Prüfung:

60 Minuten vor Prüfsubstanz Applikation wird der spezifische CB1 Antagonist *SR 141716A*, der Kontrollgruppe nur das Lösemittel (Solutol/0,9% NaCl) intraperitoneal appliziert. Die basale Körpertemperatur wird fünf Minuten vor Applikation von *SR 141716A* via Oesophagus Temperatursonde gemessen. Das weitere Vorgehen entspricht der Methode "Agonismus Prüfung". Die Gruppengröße pro Dosis beträgt 5-7 Tiere (Ratten).

### Permanente focale cerebrale Ischämie bei der Ratte (MCA-O)

Unter Isofluran Anästhesie wird die Arteria cerebri media einseitig freipräpariert und mittels Elektrokoagulation diese und deren Nebenäste irreversibel verschlossen. Als Folge des Eingriffs entsteht ein cerebraler Infarkt. Während der Operation wird die Körpertemperatur des Tieres auf 37°C gehalten. Nach Wundverschluß und Abklingen der Narkose werden die Tiere wieder in ihren Käfig entlassen. Die Substanzapplikation erfolgt nach unterschiedlichen zeitlichen Schemata und über unterschiedliche Applikationswege (i.v. i.p.) nach der Okklusion. Die Infarktgröße wird nach 7 Tagen bestimmt. Dazu wird das Gehirn entnommen, histologisch aufgearbeitet und mit Hilfe eines computergestützten Auswertsystemes das Infarktvolumen bestimmt.

| **Beispiel-Nr.** | **% Reduktion des Infarktvolumens** | **Dosis** ^{**a**)} |
|---|---|---|
| 1 | 31 | 1,0 mg/kg |
| 5 | 48 | 0,01 mg /kg |
| 18 | 20 | 0,03 mg/kg |

| | | |
|---|---|---|
| a) Die Substanzgabe als intravenöse Bolusinjektionen jeweils direkt, 2 und 4 h nach der Okklusion. | | |

### Subdurales Hämaton bei der Ratte (SDH)

Unter Anästhesie wird den Tieren einseitig subdural Eigenblut injiziert. Unter dem Hämatom bildet sich ein Infarkt. Die Substanzapplikation erfolgt nach unterschiedlichen zeitlichen Schemata und über unterschiedliche Applikationswege (i.v., i.p.). Die Bestimmung der Infarktgröße erfolgt wie beim Modell der Permanenten focalen Ischämie bei der Rattte (MCA-O) beschrieben

| **Beispiel-Nr.** | **% Reduktion des Infarktvolumens** | **Dosis ^{a)}** |
|---|---|---|
| 1 | 50% | 0,3 mg/kg |
| 5 | 58% | 0,001 mg/kg |

| | | |
|---|---|---|
| a) Substanzgabe als i.v. Bolusinjektionen jeweils direkt, 2 und 4 h nach dem Trauma. | | |

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formeln (I)-(Ie) enthalten, oder die aus einem oder mehreren Wirkstoffen der Formeln (I)-(Ie) bestehen, sowie Verfahren zur Herstellung dieser Zubereitungen.

Die Wirkstoffe der Formeln (I)-(Ie) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formeln (I)-(Ie) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formeln (I)-(Ie) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

## Patentansprüche

1. Verwendung von bekannten Agonisten des Cannabinoid Rezeptors CB1 zur Herstellung eines Arzneimittels für Prophylaxe und Behandlung von neurodegenerativen Erkrankungen, insbesondere von Apoplexia Cerebri und Schädel/Hirn-Trauma.

2. Verwendung nach Anspruch 1, worin der bekannte Agonist des Cannabinoid Rezeptors CB1 zur Prophylaxe und Behandlung von Apoplexia Cerebri eingesetzt wird.

3. Verwendung nach Anspruch 1, worin der bekannte Agonist des Cannabinoid Rezeptors CB1 zur Prophylaxe und Behandlung von Schädel/Hirn-Trauma eingesetzt wird.

4. Verwendung nach irgendeinem der Ansprüche 1 bis 3, worin der Agonist des Cannabinoid Rezeptors CB1 die allgemeinen Formel (I) aufweist: 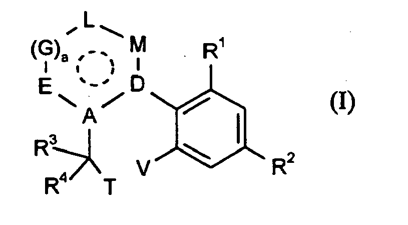 in welcher
A und D gleich oder verschieden sind und in Abhängigkeit von der Lage einer Einfach- oder Doppelbindung für ein C-Atom oder für die CH-Gruppe stehen,
E in Abhängigkeit von der Lage einer Einfach- oder Doppelbindung für die CH- oder CH₂-Gruppe oder für ein Schwefelatom steht,
G, L und M gleich oder verschieden sind und in Abhängigkeit von der Lage einer Einfach- oder Doppelbindung für einen Rest der Formel -CR⁵, -CR⁶R⁷ oder N-R⁸ stehen,
worin
R⁵, R⁶, R⁷ und R⁸ gleich oder verschieden sind und Wasserstoff, Hydroxy, Formyl, (C₂-C₆)-Alkenyl, (C₂-C₆)-Alkinyl bedeuten, oder
(C₁-C₆)-Alkyl bedeuten, das gegebenenfalls durch Hydroxy oder (C₁-C₄)-Alkoxy substituiert ist,
oder
R⁶ und R⁷ gemeinsam für einen Rest der Formel =O stehen,
a für ein Zahl 0 oder 1 steht,
R¹ für Wasserstoff oder Hydroxy steht, oder für (C₁-C₁₁)-Alkyl, (C₁-C₆)-Alkoxy, (C₁-C₆)-Alkoxycarbonyl oder (C₁-C₄)-Acyloxy steht, die gegebenenfalls durch Hydroxy, (C₁-C₁₀)-Alkoxy oder durch eine Gruppe der Formel -NR⁹R¹⁰ substituiert sind,
worin
R⁹ und R¹⁰ gleich oder verschieden sind und Wasserstoff; Phenyl, (C₁-C₄)-Alkyl bedeuten,
oder
R⁹ und R¹⁰ gemeinsam mit dem Stickstoffatom einen 5- bis 7-gliedrigen gesättigten Heterocyclus bilden, der gegebenenfalls noch ein weiteres Heteroatom aus der Reihe S und O oder einen Rest der Formel -NR¹¹ enthalten kann,
worin
R¹¹ Wasserstoff, Phenyl, (C₁-C₆)-Alkyl oder (C₁-C₆)-Acyl bedeutet,
R² für (C₁-C₁₀)-Alkyl oder (C₁-C₁₀)-Alkoxy steht, die gegebenenfalls durch Phenyl, Halogen, Hydroxy, Azido, Nitro, Trifluormethyl, Trifluormethoxy, Carboxyl, (C₁-C₄)-Alkoxy oder (C₁-C₄)-Alkoxycarbonyl oder durch eine Gruppe der Formel -NR¹²R¹³ substituiert sind,
worin
R¹² und R¹³ gleich oder verschieden sind und die oben angegebene Bedeutung von R⁹ und R¹⁰ haben,
R³ und R⁴ gleich oder verschieden sind und für Wasserstoff oder für (C₁-C₆)-Alkyl stehen, das gegebenenfalls durch Hydroxy substituiert ist,
oder
R³ und R⁴ gemeinsam für einen Rest der Formel H₂C= stehen,
T für (C₁-C₆)-Alkyl oder (C₂-C₆)-Alkenyl steht,
und
V für Hydroxy steht,
oder
T und V gemeinsam unter einem Ringschluß, für ein Sauerstoffatom oder für einen Rest der Formel -NR¹⁴ stehen,
worin
R¹⁴ Wasserstoff oder Methyl bedeutet
mit Ausnahme von Verbindungen folgender Konfiguration 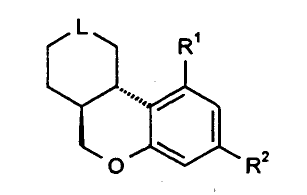 und deren Salze und isomere Formen.

5. Verwendung nach irgendeinem der Ansprüche 1 bis 3, worin der Agonist des Cannabinoid Rezeptors CB1die allgemeinen Formel (la) aufweist: 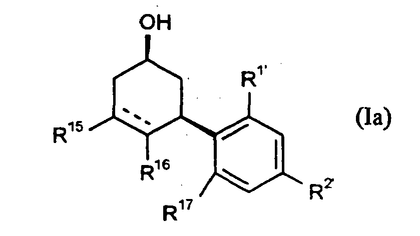 in welcher
R^{1'} und R^{2'} gleich oder verschieden sind und die im Anspruch 4 angegebene Bedeutung von R¹ und R² haben,
R¹⁵ und R¹⁶ gleich oder verschieden sind und für Wasserstoff oder (C₁-C₈)-Alkyl stehen, das gegebenenfalls durch Hydroxy substituiert ist,
oder
R¹⁵ und R¹⁶ gemeinsam unter Einbezug der C-C-Bindung einen Phenylring oder einen 3- bis 7-gliedrigen carbocyclischen Ring bilden, wobei die Ringsysteme gegebenenfalls durch (C₁-C₆)-Alkoxycarbonyl oder durch (C₁-C₈)-Alkyl substituiert sind, das seinerseits durch Hydroxy substituiert sein kann,
R¹⁷ für Wasserstoff steht,
oder
R¹⁶ und R¹⁷ gemeinsam einen 6-gliedrigen gesättigten, partiell gesättigten oder ungesättigten Heterocyclus bilden, der ein Heteroatom aus der Reihe S und O oder einen Rest der Formel -NR¹⁸ enthalten kann,
worin
R¹⁸ Wasserstoff oder (C₁-C₄)-Alkyl bedeutet,
und wobei die Ringsysteme gegebenenfalls bis zu 3-fach gleich oder verschieden, auch geminal, durch (C₁-C₈)-Alkyl substituiert sind, das seinerseits durch Hydroxy substituiert sein kann
und deren Salze und isomere Formen.

6. Verwendung nach irgendeinem der Ansprüche 1 bis 3, worin der Agonist des Cannabinoid Rezeptors CB1 ein Aminoalkylindol der allgemeinen Formel (Ib) oder (Ic) ist: 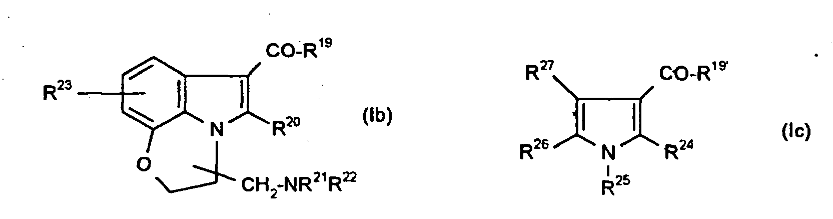 in welcher
R¹⁹ und R^{19'} gleich oder verschieden sind und für (C₆-C₁₀)-Aryl oder für einen 5- bis 7-gliedrigen, gesättigten oder ungesättigten Heterocyclus mit bis zu 3 Heteroatomen aus der Reihe S, N und/oder O stehen, die gegebenenfalls durch einen oder mehreren, gleichen oder verschiedenen Substituenten substituiert sind, die aus der Gruppe ausgewählt sind, die besteht aus: Nitro, Halogen, Trifluormethyl, Hydroxy, Carboxyl oder durch (C₁-C₅)-Acyl, (C₁-C₆)-Alkoxy, (C₁-C₆)-Alkylthio, (C₁-C₆)-Alkoxycarbonyl und (C₁-C₆)-Alkyl, das seinerseits durch Hydroxy substituiert sein kann,
R²⁰ für Wasserstoff oder Methyl steht,
R²¹ und R²² gleich oder verschieden sind und für Wasserstoff oder für (C₁-C₆)-Alkyl stehen, oder
R²¹ und R²² gemeinsam mit dem Stickstoffatom einen 5- bis 7-gliedrigen, gesättigten oder partiell gesättigten Heterocyclus bilden, der gegebenenfalls ein weiteres Sauerstoff- oder Schwefelatom oder einen Rest der Formel -NR²⁸ enthalten kann,
worin
R²⁸ die oben angegebene Bedeutung von R⁸ hat und mit dieser gleich oder verschieden ist,
R²³ für Wasserstoff, Halogen, Hydroxy, (C₁-C₈)-Alkyl oder für (C₁-C₈)-Alkoxy steht,
R²⁴ für Wasserstoff oder für (C₁-C₆)-Alkyl steht,
R²⁵ für Wasserstoff, Phenyl, Cycloalkyl mit 3 bis 8 Kohlenstoffatomen, oder für (C₁-C₆)-Alkyl steht, das gegebenenfalls durch eine Gruppe der Formel -NR²⁹R³⁰ substituiert ist,
worin
R²⁹ und R³⁰ gleich oder verschieden sind und die oben angegebene Bedeutung von R⁹ und R¹⁰ haben ,
R²⁶ und R²⁷ für Wasserstoff stehen, oder gemeinsam unter Einbezug der Doppelbindung einen Phenylring bilden
und deren Salze und isomere Formen.

7. Verwendung nach irgendeinem der Ansprüche 1 bis 3, worin der Agonist des Cannabinoid Rezeptors CB1 die allgemeinen Formel (Id) aufweist: 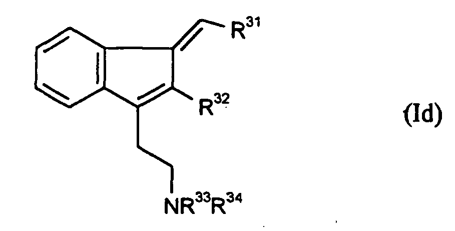 in welcher
R³¹ die oben angegebene Bedeutung von R¹⁹ hat und mit dieser gleich oder verschieden ist,
R³² für Wasserstoff, (C₁-C₃)-Alkyl oder für (C₁-C₃)-Alkoxy steht,
R³³ und R³⁴ die oben angegebene Bedeutung von R²¹ und R²² haben und mit dieser gleich oder verschieden sind
und deren Salze und isomere Formen.

8. Verwendung nach irgendeinem der Ansprüche 1 bis 3, worin der Agonist des Cannabinoid Rezeptors CB1 ein Eicosanoid der allgemeinen Formel (Ie) ist: 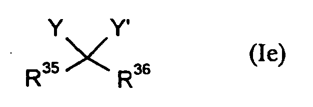 in welcher
Y und Y' für Wasserstoff stehen,
oder
Y und Y' gemeinsam für einen Rest der Formel =O oder =S stehen,
R³⁵ für (C₁₆-C₃₀)-Alkenyl mit mindestens drei Doppelbindungen steht,
R³⁶ für Trifluormethyl oder für eine Gruppe der Formel -OR³⁷ oder -NR³⁸R³⁹ steht,
worin
R³⁷ Wasserstoff oder (C₁-C₁₀)-Alkyl bedeutet, das gegebenenfalls mit einem oder mehreren, gleichen oder verschiedenen Substituenten substituiert ist, die aus der Gruppe ausgewählt sind, die besteht aus: Hydroxy, Halogen, Trifluormethyl, (C₆-C₁₀)-Aryl und (C₁-C₆)-Alkoxy,
R³⁸ und R³⁹ die oben angegebene Bedeutung von R³⁷ haben und mit dieser gleich oder verschieden sind,
oder
R³⁸ und R³⁹ gemeinsam mit dem Stickstoffatom einen 5- bis 7-gliedrigen gesättigten Heterocyclus bilden, der gegebenenfalls ein weiteres Heteroatom aus der Reihe S und O oder eine Gruppe der Formel -NR⁴⁰ enthalten kann,
worin
R⁴⁰ die oben angegebene Bedeutung von R⁸ hat und mit dieser gleich oder verschieden ist,
und deren Salze und isomere Formen.

9. Verwendung nach irgendeinem der Ansprüche 1 bis 3, worin der Agonist des Cannabinoid Rezeptors CB1 aus Verbindungen der folgenden Formeln ausgewählt wird:

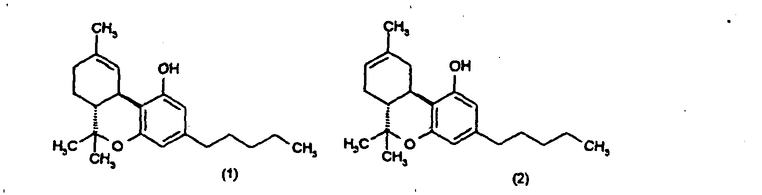

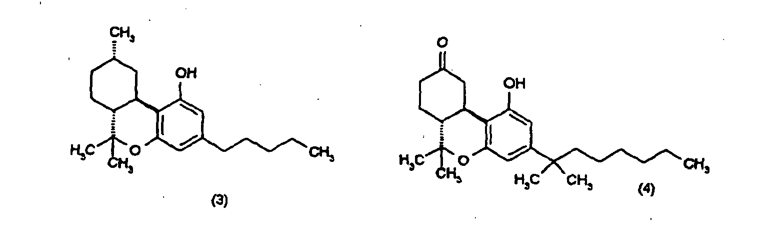

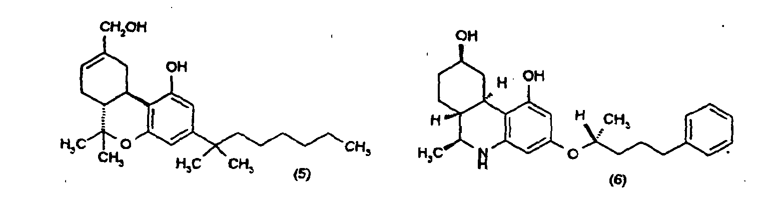

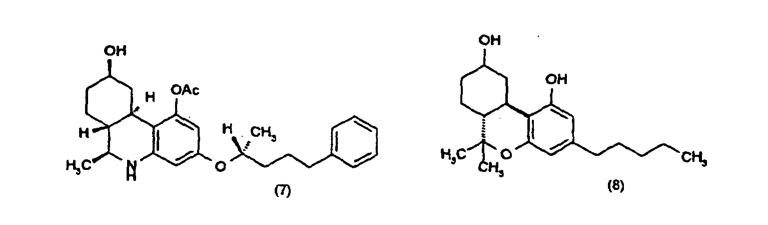

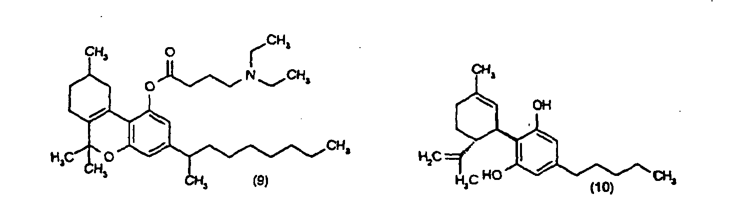

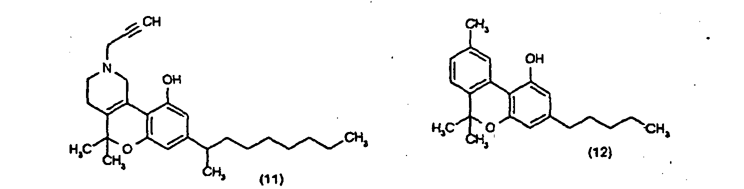

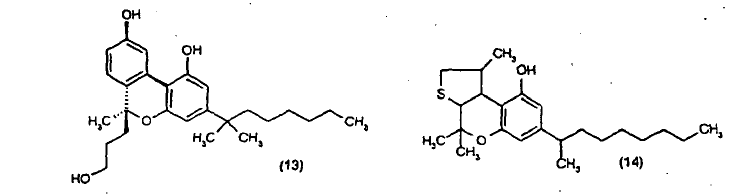

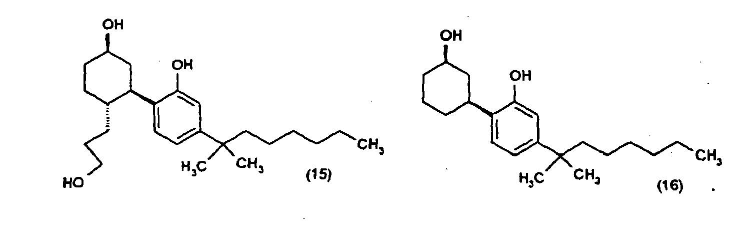

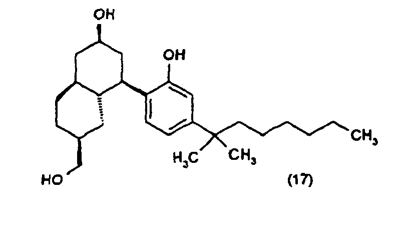

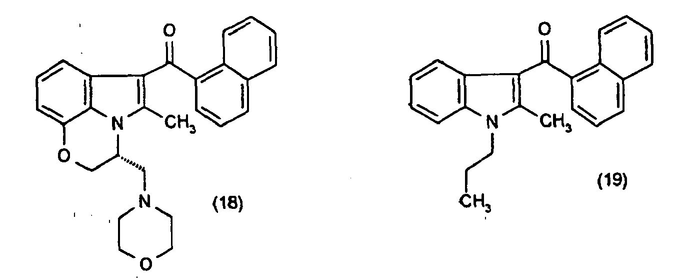

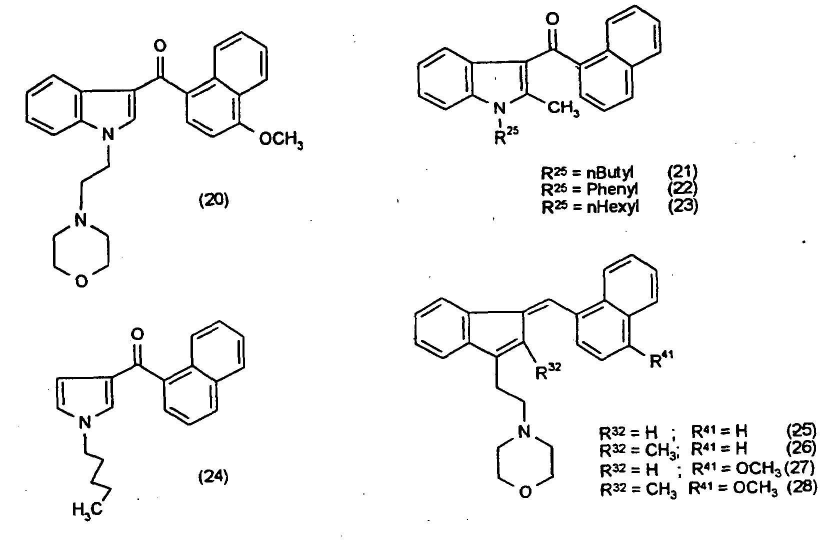

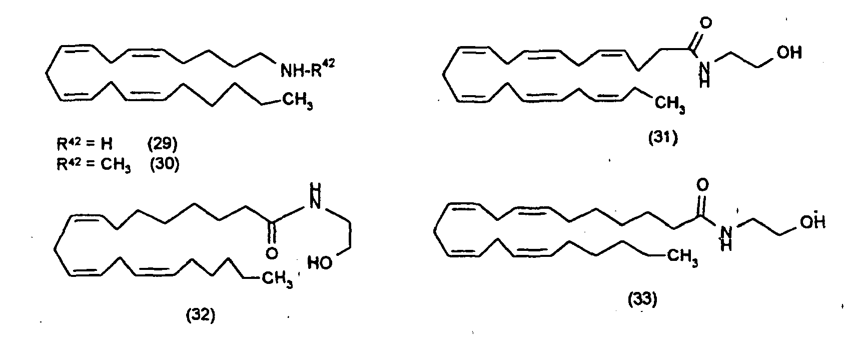

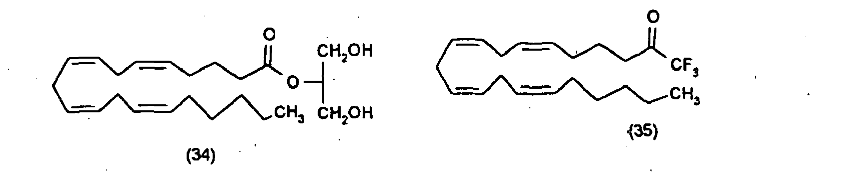

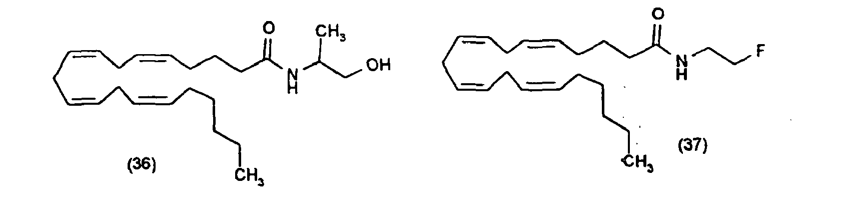

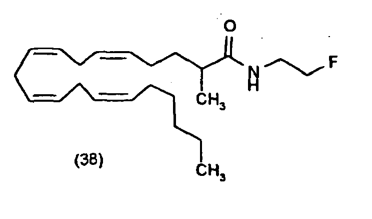

10. Verwendung nach Anspruch 9, worin der Agonist des Cannabinoid Rezeptors CB1 aus Verbindungen der folgenden Formeln ausgewählt wird:

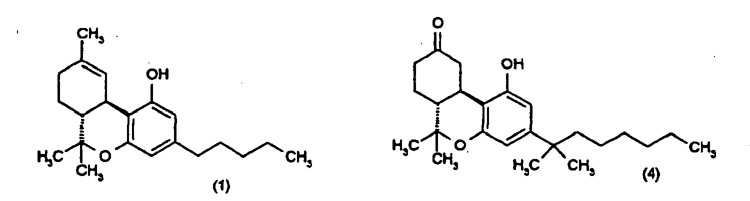

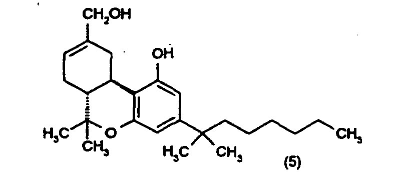

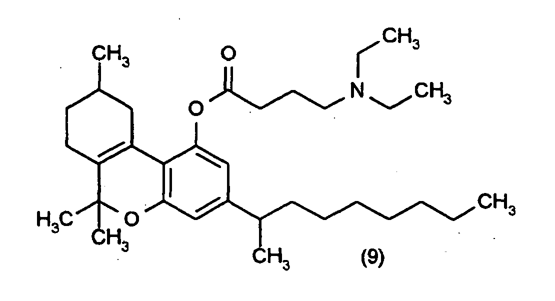

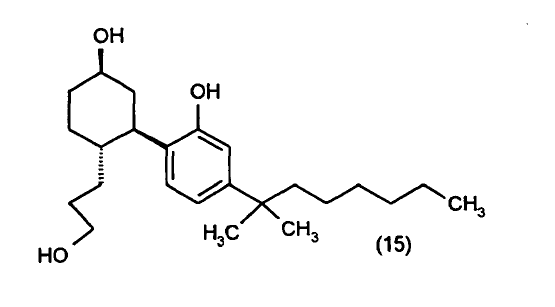

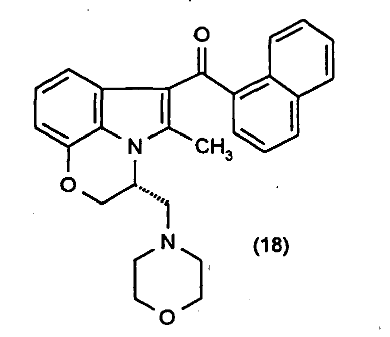

## Claims

1. Use of known agonists of the cannabinoid receptor CB1 for the preparation of a medicament for the prophylaxis and treatment of neurodegenerative disorders, in particular of cerebral apoplexy and craniocerebral trauma.

2. Use according to Claim 1, in which the known agonist of the cannabinoid receptor CB1 is employed for the prophylaxis and treatment of cerebral apoplexy.

3. Use according to Claim 1, in which the known agonist of the cannabinoid receptor CB1 is employed for the prophylaxis and treatment of craniocerebral trauma.

4. Use according to any of Claims 1 to 3, in which the agonist of the cannabinoid receptor CB1 has the general formula I: 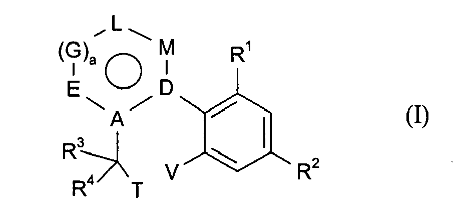 in which
A and D are identical or different and, depending on the position of a single or double bond, represent a C atom or the CH group,
E depending on the position of a single or double bond, represents the CH or CH₂ group or a sulphur atom,
G, L and M are identical or different and, depending on the position of a single or double bond, represent a radical of the formula -CR⁵, -CR⁶R⁷ or N-R⁸,
in which
R⁵, R⁶, R⁷ and R⁸ are identical or different and denote hydrogen, hydroxyl, formyl, (C₂-C₆)-alkenyl, (C₂-C₆)-alkinyl, or
denote (C₁-C₆)-alkyl which is optionally substituted by hydroxyl or (C₁-C₄)-alkoxy,
or
R⁶ and R⁷ together represent a radical of the formula =O,
a represents a number 0 or 1,
R¹ represents hydrogen or hydroxyl, or represents (C₁-C₁₁)-alkyl, (C₁-C₆)-alkoxy, (C₁-C₆)-alkoxycarbonyl or (C₁-C₄)-acyloxy, each of which is optionally substituted by hydroxyl, (C₁-C₁₀)-alkoxy or by a group of the formula -NR⁹R¹⁰,
in which
R⁹ and R¹⁰ are identical or different and denote hydrogen, phenyl, (C₁-C₄)-alkyl,
or
R⁹ and R¹⁰, together with the nitrogen atom, form a 5- to 7-membered saturated heterocycle which can optionally additionally contain a further heteroatom of the series S and O or a radical of the formula -NR¹¹,
in which
R¹¹ denotes hydrogen, phenyl, (C₁-C₆)-alkyl or (C₁-C₆)-acyl,
R² represents (C₁-C₁₀)-alkyl or (C₁-C₁₀)-alkoxy, each of which is optionally substituted by phenyl, halogen, hydroxyl, azido, nitro, trifluoromethyl, trifluoromethoxy, carboxyl, (C₁-C₄)-alkoxy or (C₁-C₄)-alkoxycarbonyl or by a group of the formula -NR¹²R¹³,
in which
R¹² and R¹³ are identical or different and have the meaning of R⁹ and R¹⁰ indicated above,
R³ and R⁴ are identical or different and represent hydrogen or (C₁-C₆)-alkyl which is optionally substituted by hydroxyl,
or
R³ and R⁴ together represent a radical of the formula H₂C=,
T represents (C₁-C₆)-alkyl or (C₂-C₆)-alkenyl,
and
V represents hydroxyl,
or
T and V together, with a ring closure, represent an oxygen atom or a radical of the formula -NR¹⁴,
in which
R¹⁴ denotes hydrogen or methyl
with the exception of compounds of the following configuration 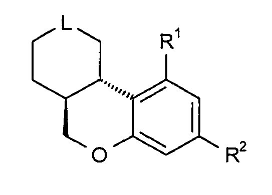 and their salts and isomeric forms.

5. Use according to any of Claims 1 to 3, in which the agonist of the cannabinoid receptor CB1 has the general formula (Ia): 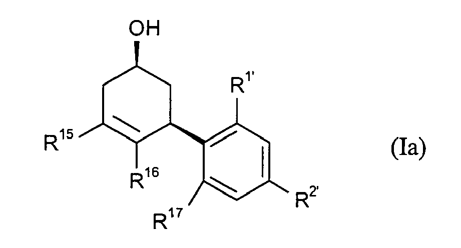 in which
R^{1'} and R^{2'} are identical or different and have the meaning of R¹ and R² indicated in Claim 4,
R¹⁵ and R¹⁶ are identical or different and represent hydrogen or (C₁-C₈)-alkyl which is optionally substituted by hydroxyl,
or
R¹⁵ and R¹⁶ together, with inclusion of the C-C bond, form a phenyl ring or a 3- to 7-membered carbocyclic ring, where the ring systems are optionally substituted by (C₁-C₆)-alkoxycarbonyl or (C₁-C₈)-alkyl which for its part can be substituted by hydroxyl,
R¹⁷ represents hydrogen,
or
R¹⁶ and R¹⁷ together form a 6-membered saturated, partially saturated or unsaturated heterocycle which can contain a heteroatom of the series S and O or a radical of the formula -NR¹⁸,
in which
R¹⁸ denotes hydrogen or (C₁-C₄)-alkyl,
and where the ring systems are optionally substituted up to 3 times identically or differently, also geminally, by (C₁-C₈)-alkyl which for its part can be substituted by hydroxyl,
and their salts and isomeric forms.

6. Use according to any of Claims 1 to 3, in which the agonist of the cannabinoid receptor CB1 is an aminoalkylindole of the general formula (Ib) or (Ic): 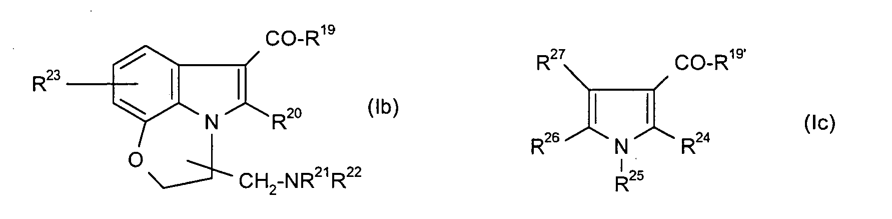 in which
R¹⁹ and R^{19'} are identical or different and represent (C₆-C₁₀)-aryl or a 5- to 7-membered, saturated or unsaturated heterocycle having up to 3 heteroatoms of the series S, N and/or O, each of which is optionally substituted by one or more, identical or different substituents which are selected from the group which consists of: nitro, halogen, trifluoromethyl, hydroxyl, carboxyl, or by (C₁-C₅)-acyl, (C₁-C₆)-alkoxy, (C₁-C₆)-alkylthio, (C₁-C₆)-alkoxycarbonyl and (C₁-C₆)-alkyl which for its part can be substituted by hydroxyl,
R²⁰ represents hydrogen or methyl,
R²¹ and R²² are identical or different and represent hydrogen or (C₁-C₆)-alkyl, or
R²¹ and R²², together with the nitrogen atom, form a 5- to 7-membered, saturated or partially saturated heterocycle which can optionally contain a further oxygen or sulphur atom or a radical of the formula -NR²⁸,
in which
R²⁸ has the meaning of R⁸ indicated above and is identical to or different from this,
R²³ represents hydrogen, halogen, hydroxyl, (C₁-C₈)-alkyl or (C₁-C₈)-alkoxy,
R²⁴ represents hydrogen or (C₁-C₆)-alkyl,
R²⁵ represents hydrogen, phenyl, cycloalkyl having 3 to 8 carbon atoms, or (C₁-C₆)-alkyl which is optionally substituted by a group of the formula -N²⁹R³⁰,
in which
R²⁹ and R³⁰ are identical or different and have the meaning of R⁹ and R¹⁰ indicated above,
R²⁶ and R²⁷ represent hydrogen, or Together, with inclusion of the double bond, form a phenyl ring
and their salts and isomeric forms.

7. Use according to any of Claims 1 to 3, in which the agonist of the cannabinoid receptor CB1 has the general formula (Id): 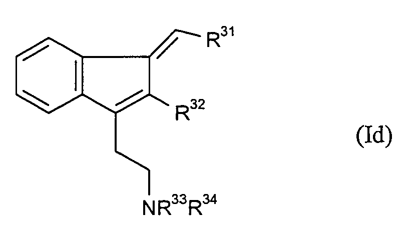 in which
R³¹ has the meaning of R¹⁹ indicated above and is identical to or different from this,
R³² represents hydrogen, (C₁-C₃)-alkyl or (C₁-C₃)-alkoxy,
R³³ and R³⁴ have the meaning of R²¹ and R²² indicated above and are identical to or different from this
and their salts and isomeric forms.

8. Use according to any of Claims 1 to 3, in which the agonist of the cannabinoid receptor CB1 is an eicosanoid of the general formula (Ie): 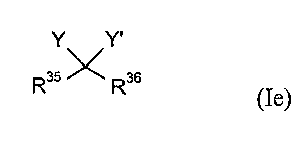 in which
Y and Y' represent hydrogen,
or
Y and Y' together represent a radical of the formula =O or =S,
R³⁵ represents (C₁₆-C₃₀)-alkenyl having at least three double bonds,
R³⁶ represents trifluoromethyl or a group of the formula -OR³⁷ or -NR³⁸R³⁹,
in which
R³⁷ denotes hydrogen or (C₁-C₁₀)-alkyl which is optionally substituted by one or more, identical or different substituents which are selected from the group which consists of: hydroxyl, halogen, trifluoromethyl, (C₆-C₁₀)-aryl and (C₁-C₆)-alkoxy,
R³⁸ nd R³⁹ a have the meaning of R³⁷ indicated above and are identical to or different from this,
or
R³⁸ and R³⁹, together with the nitrogen atom, form a 5- to 7-membered saturated heterocycle which can optionally contain a further heteroatom of the series S and O or a group of the formula -NR⁴⁰,
in which
R⁴⁰ has the meaning of R⁸ indicated above and is identical to or different from this,
and their salts and isomeric forms.

9. Use according to any of Claims 1 to 3, in which the agonist of the cannabinoid receptor CB1 is selected from compounds of the following formulae:

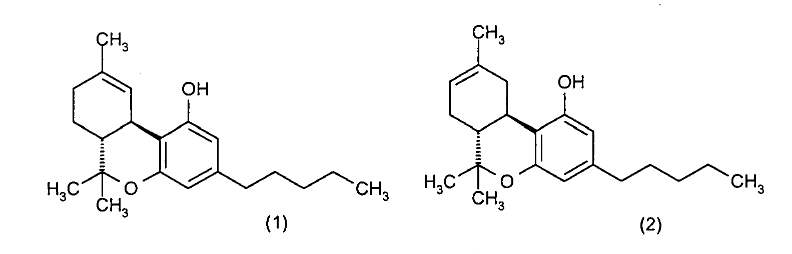

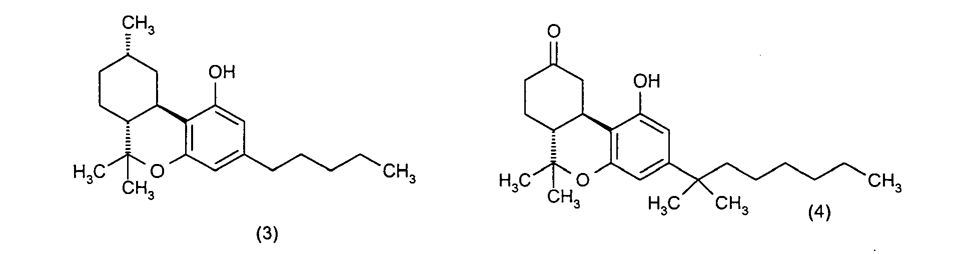

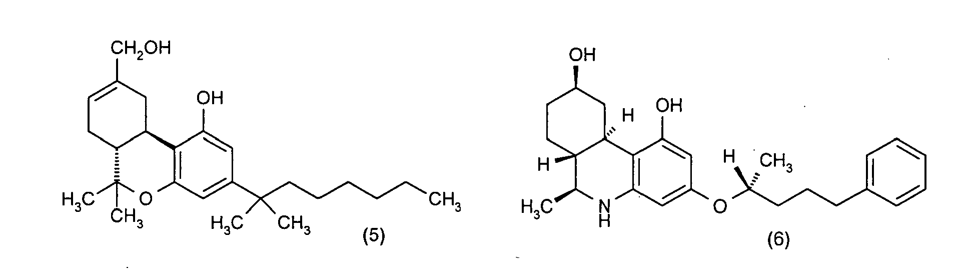

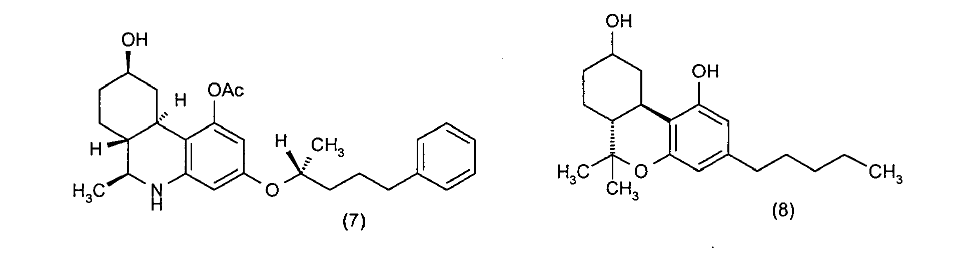

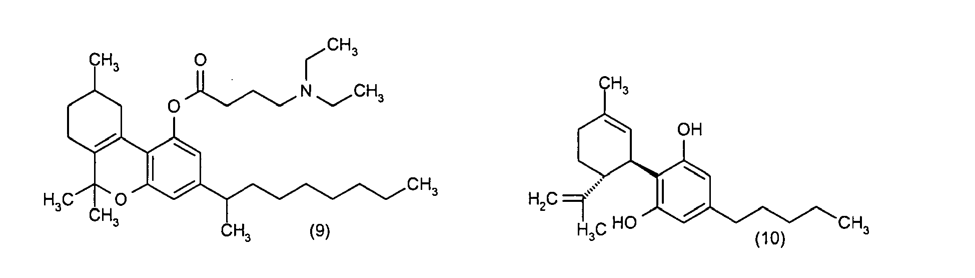

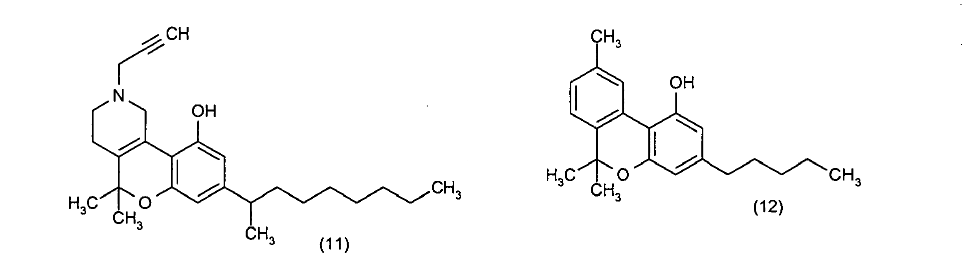

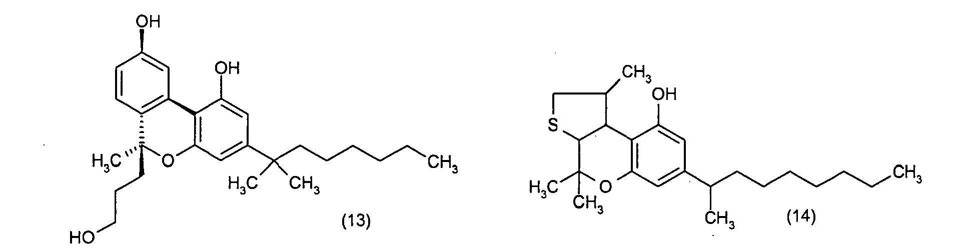

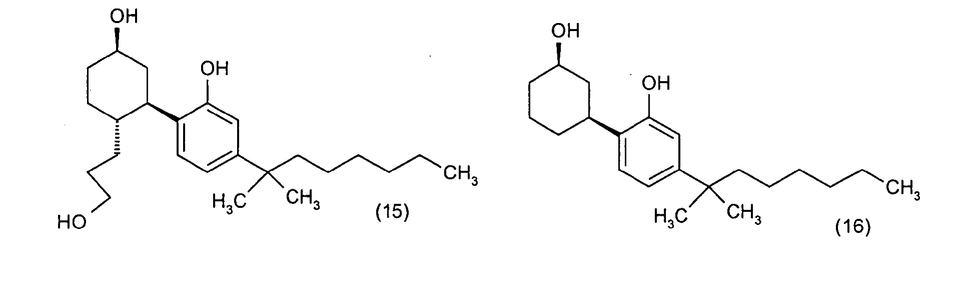

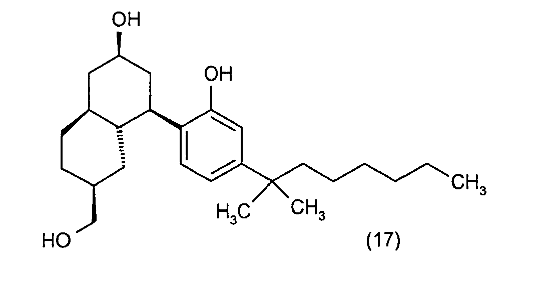

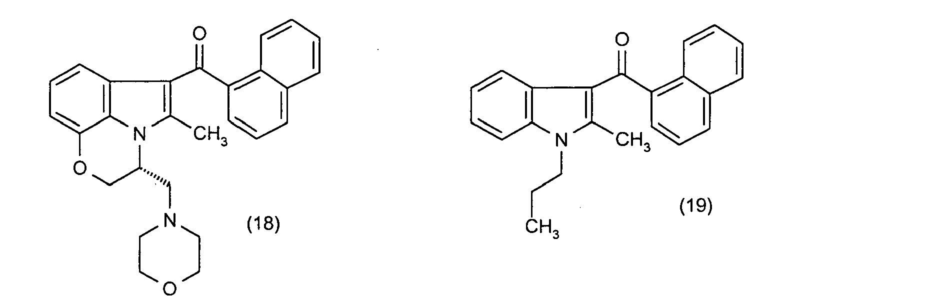

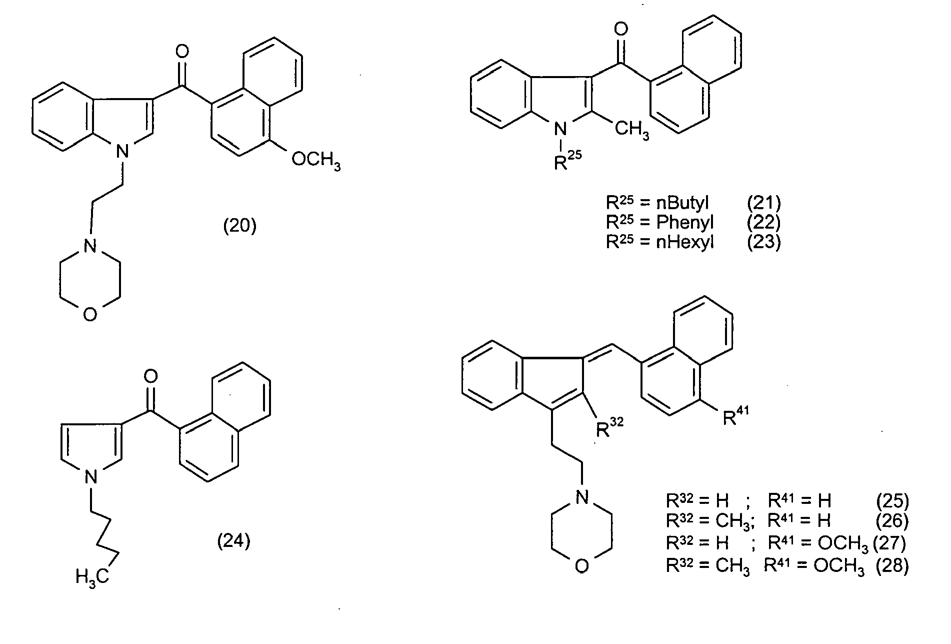

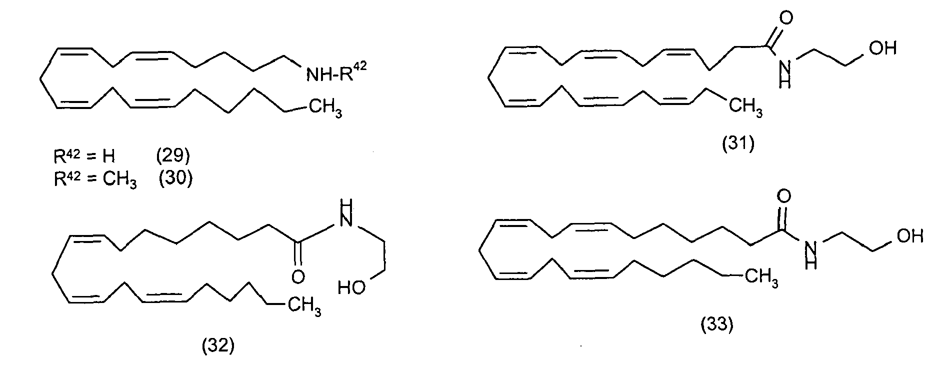

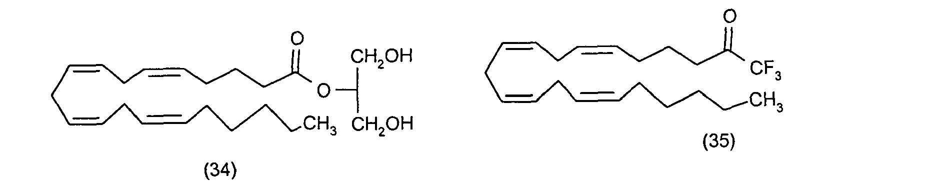

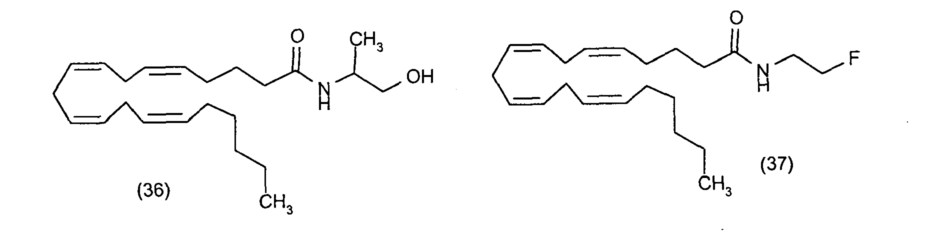

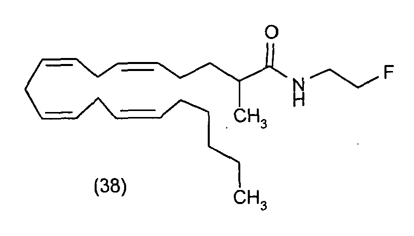

10. Use according to Claim 9, in which the agonist of the cannabinoid receptor CB1 is selected from compounds of the following formulae:

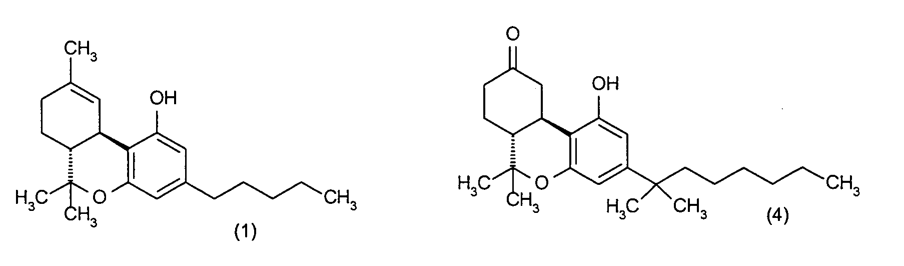

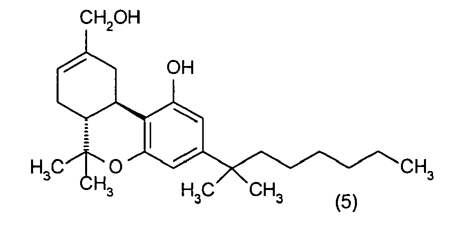

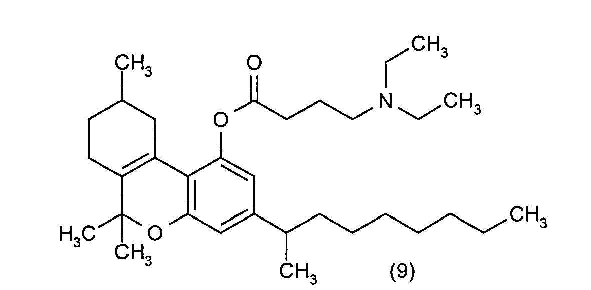

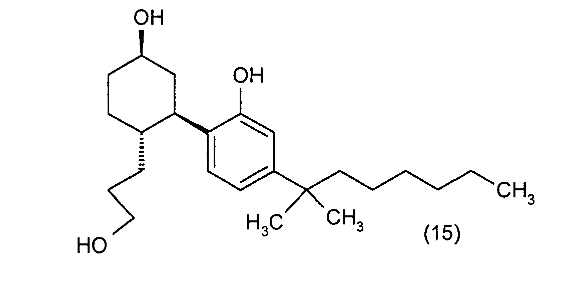

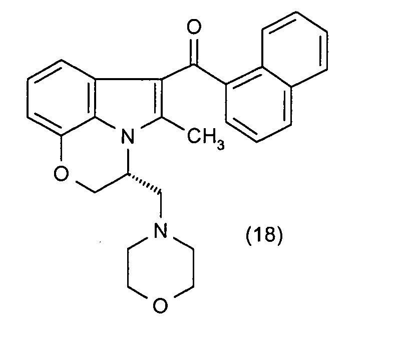

## Revendications

1. Utilisation d'agonistes connus du récepteur cannabinoïde CB1 pour la préparation d'un médicament destiné à la prophylaxie et au traitement de maladies neurodégénératives, en particulier de l'apoplexie cérébrale et du traumatisme craniocérébral.

2. Utilisation suivant la revendication 1, dans laquelle l'agoniste connu du récepteur cannabinoïde CB1 est utilisé pour la prophylaxie et le traitement de l'apoplexie cérébrale.

3. Utilisation suivant la revendication 1, dans laquelle l'agoniste connu du récepteur cannabinoïde CB1 est utilisé pour la prophylaxie et le traitement du traumatisme craniocérébral.

4. Utilisation suivant l'une quelconque des revendications 1 à 3, dans laquelle l'agoniste du récepteur cannabinoïde CB1 répond à la formule générale (1) : 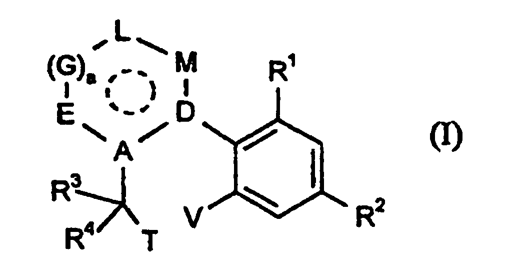 dans laquelle
A et D sont identiques ou différents et représentent, en fonction de la position d'une liaison simple ou d'une
E liaison double, un atome de carbone ou le groupe CH, représente, en fonction de la position d'une liaison simple ou d'une liaison double, le groupe CH ou CH₂ ou un atome de soufre,
G, L et M sont identiques ou différents et représentent, en fonction de la position d'une liaison simple ou d'une liaison double, un reste de formule -CR⁵, -CR⁶R⁷ ou N-R⁸,
où
R⁵, R⁶, R⁷ et R⁸ sont identiques ou différents et représentent l'hydrogène, un groupe hydroxy, formyle, alcényle en C₂ à C₆, alcynyle en C₂ à C₆,
ou bien
ils représentent un groupe alkyle en C₁ à C₆ qui est éventuellement substitué par un radical hydroxy ou alkoxy en C₁ à C₄,
ou bien
R⁶ et R⁷ forment ensemble un reste de formule =O,
a représente le nombre 0 ou 1,
R¹ représente l'hydrogène ou un groupe hydroxy, ou bien un groupe alkyle en C₁ à C₁₁, alkoxy en C₁ à C₆, (alkoxy en C₁ à C₆)carbonyle ou un groupe acyloxy en C₁ à C₄, ces groupes étant éventuellement substitués par un radical hydroxy, alkoxy en C₁ à C₁₀ ou par un groupe de formule -NR⁹R¹⁰,
dans laquelle
R⁹ et R¹⁰ sont identiques ou différents et représentent l'hydrogène, un groupe phényle, alkyle en C₁ à C₄,
ou bien
R⁹ et R¹⁰ forment, conjointement avec l'atome d'azote, un hétérocycle saturé pentagonal à heptagonal qui peut éventuellement contenir encore un autre hétéroatome de la série S et O ou un reste de formule -NR¹¹,
dans laquelle
R¹¹ représente l'hydrogène, un groupe phényle, alkyle en C₁ à C₆ ou acyle en C₁ à C₆,
R² représente un groupe alkyle en C₁ à C₁₀ ou un groupe alkoxy en C₁ à C₁₀, qui est éventuellement substitué par un radical phényle, halogéno, hydroxy, azido, nitro, trifluorométhyle, trifluorométhoxy, carboxyle, alkoxy en C₁ à C₉ ou (alkoxy en C₁ à C₉) carbonyle ou par un groupe de formule -NR¹²R¹³,
dans laquelle
R¹² et R¹³ sont identiques ou différents et ont la définition indiquée ci-dessus pour R⁹ et R¹⁰,
R³ et R⁴ sont identiques ou différents et représentent l'hydrogène ou un groupe alkyle en C₁ à C₆ qui est éventuellement substitué par un radical hydroxy,
ou bien
R³ et R⁹ forment ensemble un reste de formule H₂C=,
T est un groupe alkyle en C₁ à C₆ ou alcényle en C₂ à C₆, et
V est un groupe hydroxy,
ou bien
T et V s'associent en formant une cyclisation, un atome d'oxygène ou un reste de formule -NR¹⁴,
où
R¹⁴ représente l'hydrogène ou un reste méthyle,
à l'exception de composés de configuration suivante : 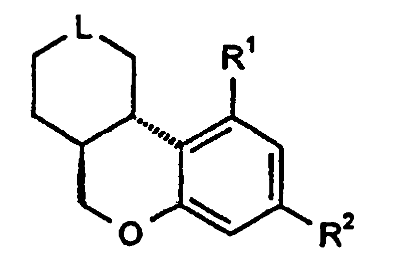 et leurs sels et leurs formes isomères.

5. Utilisation suivant l'une quelconque des revendications 1 à 3, dans laquelle l'agoniste du récepteur cannabinoïde CB1 répond à la formule générale (Ia) : 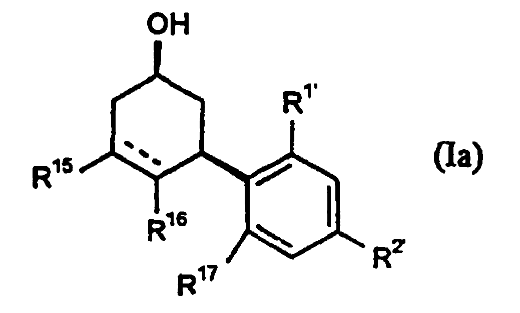 dans laquelle
R^{1'} et R^{2'} sont identiques ou différents et ont la définition indiquée pour R¹ et R² dans la revendication 4,
R¹⁵ et R¹⁶ sont identiques ou différents et représentent l'hydrogène ou un groupe alkyle en C₁ à C₈ qui est éventuellement substitué par un radical hydroxy,
ou bien
R¹⁵ et R¹⁶ forment ensemble, en faisant intervenir la liaison C-C, un noyau phényle ou un noyau carbocyclique trigonal à heptagonal, les systèmes cycliques étant éventuellement substitués par un reste (alkoxy en C₁ à C₆)carbonyle ou par un reste alkyle en C₁ à C₈ qui peut lui-même être substitué par un radical hydroxy,
R¹⁷ représente l'hydrogène,
ou bien
R¹⁶ et R¹⁷ forment ensemble un hétérocycle hexagonal saturé, partiellement saturé ou insaturé, qui peut contenir un hétéroatome de la série S et 0 ou un reste de formule -NR¹⁸,
dans laquelle
R¹⁸ représente l'hydrogène ou un reste alkyle en C₁ à C₄,
les systèmes cycliques étant éventuellement substitués jusqu'à trois fois identiques ou différentes, également en configuration géminée, par un reste alkyle en C₁ à C₈ qui peut lui-même être substitué par un radical hydroxy, et leurs sels et leurs formes isomères.

6. Utilisation selon l'une quelconque des revendications 1 à 3, dans laquelle l'agoniste du récepteur cannabinoïde CB1 est un aminoalkylindole de formule générale (Ib) ou (Ic) : 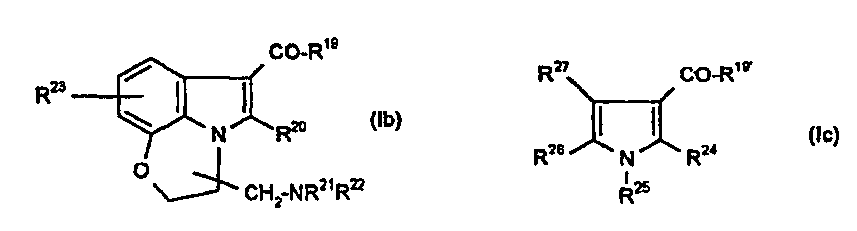 dans laquelle
R¹⁹ et R^{19'} sont identiques ou différents et représentent un reste aryle en C₆ à C₁₀ ou un hétérocycle pentagonal à heptagonal saturé ou non saturé ayant jusqu'à 3 hétéroatomes de la série S, N et/ou O, qui sont éventuellement substitués par un ou plusieurs substituants identiques ou différents qui sont choisis dans le groupe constitué de : nitro, halogéno, trifluorométhyle, hydroxy, carboxyle ou par un radical acyle en C₁ à C₅, alkoxy en C₁ à C₆, alkylthio en C₁ à C₆, (alkoxy en C₁ à C₆) carbonyle et alkyle en C₁ à C₆ qui peut lui-même être substitué par un radical hydroxy,
R²⁰ représente l'hydrogène ou un reste méthyle,
R²¹ et R²² sont identiques ou différents et représentent l'hydrogène ou un groupe alkyle en C₁ à C₆, ou bien
R²¹ et R²² forment, conjointement avec l'atome d'azote, l'hétérocycle pentagonal à heptagonal saturé ou partiellement saturé, qui peut éventuellement contenir un autre atome d'oxygène ou de soufre ou un reste de formule -NR²⁸,
où
R²⁸ a la définition indiquée ci-dessus pour R⁸ et y est identique ou en est différent,
R²³ représente l'hydrogène, un halogène, un groupe hydroxy, alkyle en C₁ à C₈ ou alkoxy en C₁ à C₈,
R²⁴ représente l'hydrogène ou un groupe alkyle en C₁ à C₆,
R²⁵ représente l'hydrogène, un groupe phényle, cycloalkyle ayant 3 à 8 atomes de carbone ou un groupe alkyle en C₁ à C₆ qui est éventuellement substitué par un groupe de formule -NR²⁹R³⁰,
où
R²⁹ et R³⁰ sont identiques ou différents et ont la définition indiquée ci-dessus pour R⁹ et R¹⁰,
R²⁶ et R²⁷ représentent l'hydrogène, ou forment ensemble un noyau phényle en incluant la double liaison,
et leurs sels et leurs formes isomères.

7. Utilisation suivant l'une quelconque des revendications 1 à 3, dans laquelle l'agoniste du récepteur cannabinoïde CB1 répond à la formule générale (Id) : 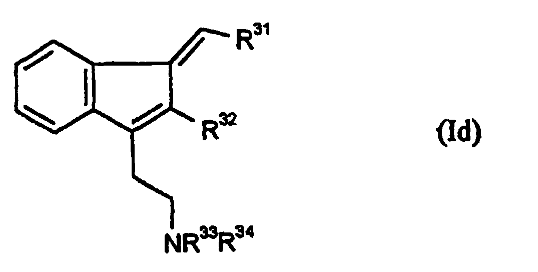 dans laquelle
R³¹ a la définition indiquée ci-dessus pour R¹⁹ et y est identique ou en est différent,
R³² représente l'hydrogène, un groupe alkyle en C₁ à C₃ ou un groupe alkoxy en C₁ à C₃,
R³³ et R³⁴ ont la définition indiquée ci-dessus pour R²¹ et R²² et y sont identiques ou en sont différents,
et leurs sels et leurs formes isomères.

8. Utilisation suivant l'une quelconque des revendications 1 à 3, dans laquelle l'agoniste du récepteur cannabinoïde CB1 est un eicosanoïde de formule générale (Ie) : 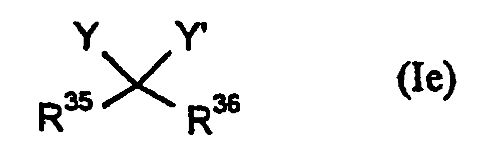 dans laquelle
Y et Y' représentent l'hydrogène,
ou bien
Y et Y' forment ensemble un reste de formule =0 ou =S,
R³⁵ est un groupe alcényle en C₁₆ à C₃₀ ayant au moins trois doubles liaisons,
R³⁶ est un groupe trifluorométhyle ou un groupe de formule -OR³⁷ ou -NR³⁸R³⁹
où
R³⁷ représente l'hydrogène ou un groupe alkyle en C₁ à C₁₀, qui est éventuellement substitué avec un ou plusieurs substituants identiques ou différents qui sont choisis dans le groupe constitué de : hydroxy, halogène, trifluorométhyle, aryle en C₆ à C₁₀ ou alkoxy en C₁ à C₆,
R³⁸ et R³⁹ ont la définition indiquée ci-dessus pour R ³⁷ et y sont identiques ou en sont différents,
ou bien
R³⁸ et R³⁹ forment, conjointement avec l'atome d'azote, un hétérocycle saturé pentagonal à heptagonal qui peut éventuellement contenir un autre hétéroatome de la série S et 0 ou un groupe de formule -NR⁴⁰
dans laquelle
R⁴⁰ a la définition indiquée ci-dessus pour R⁸ et y est identique ou en est différent,
et leurs sels et leurs formes isomères.

9. Utilisation suivant l'une quelconque des revendications 1 à 3, dans laquelle l'agoniste du récepteur cannabinoïde CB1 est choisi entre des composés de formules suivantes :

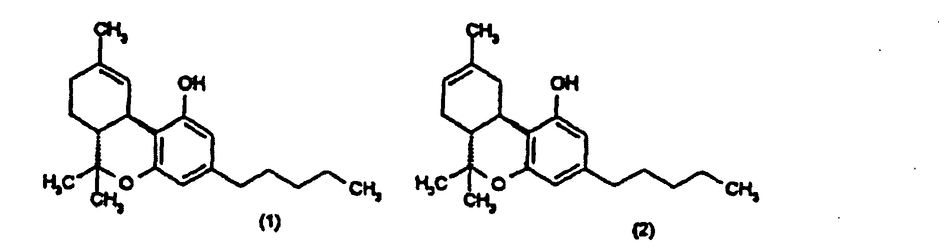

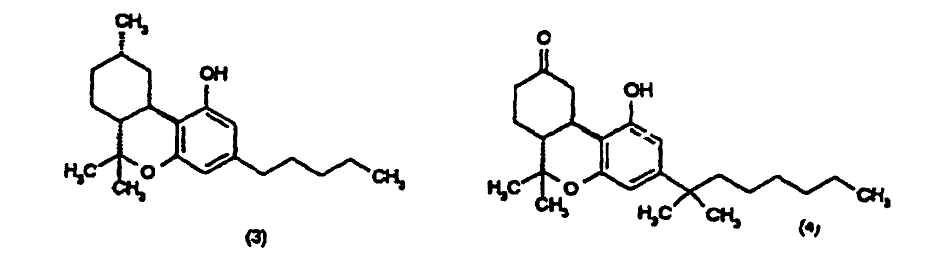

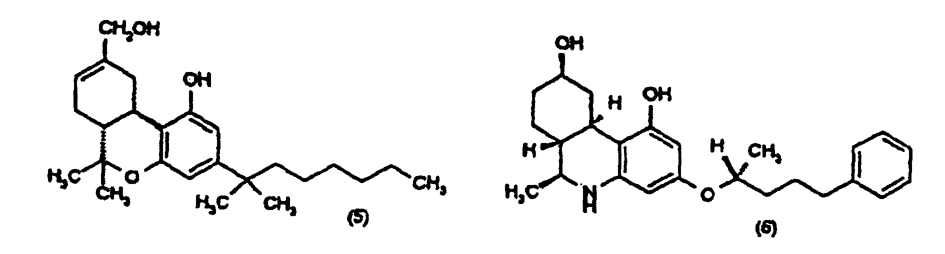

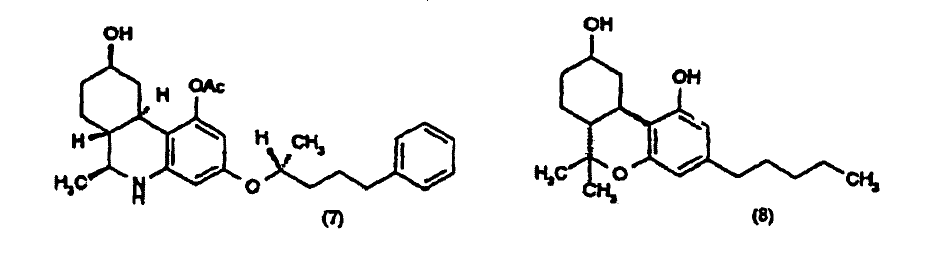

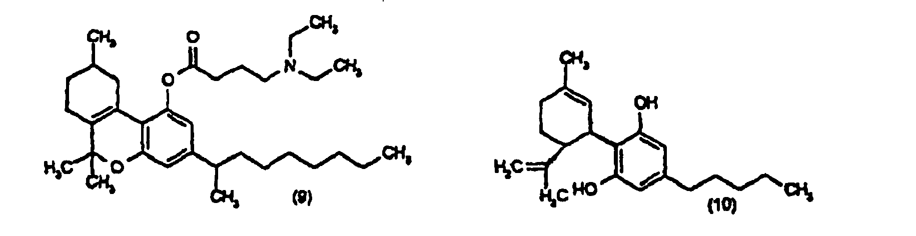

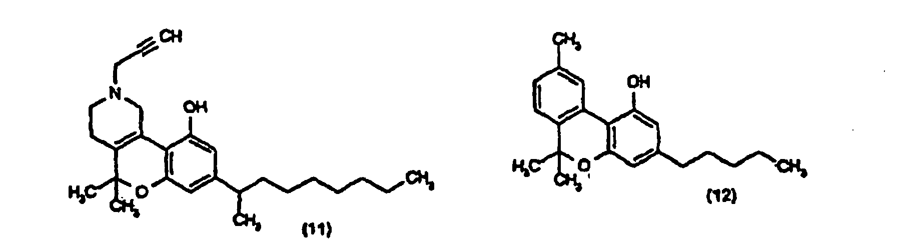

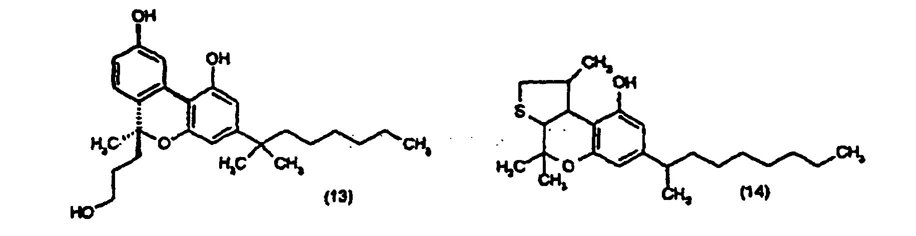

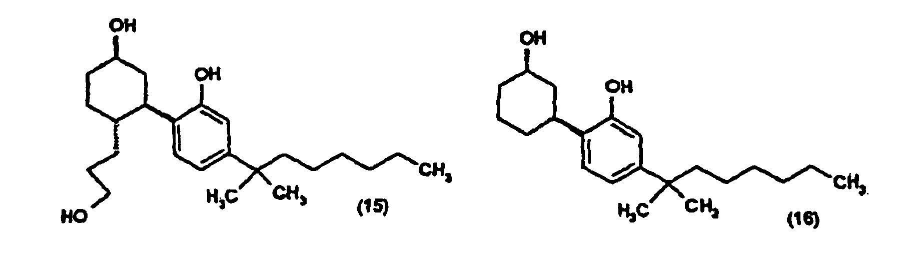

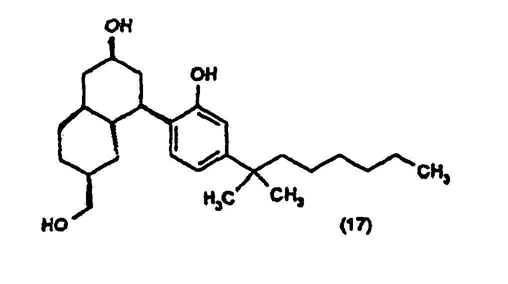

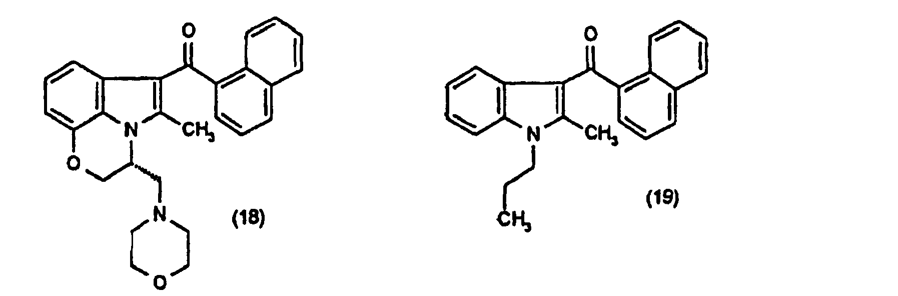

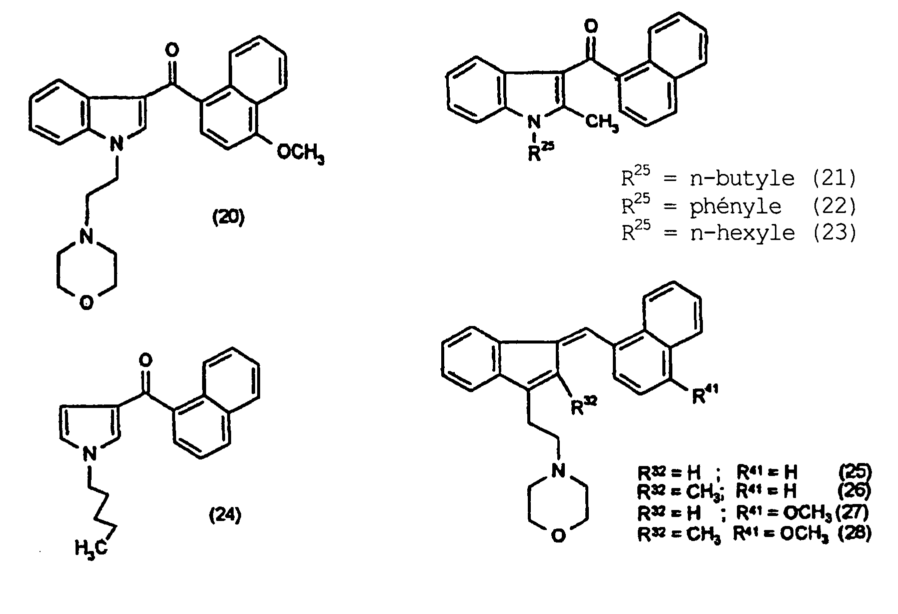

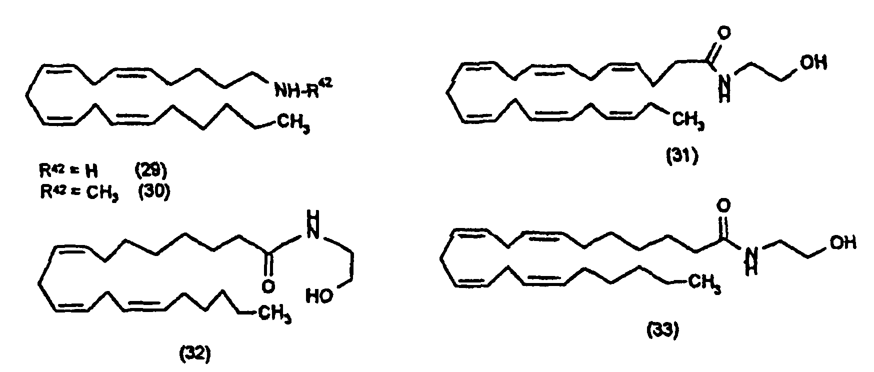

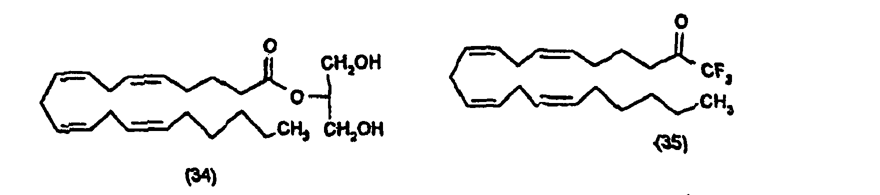

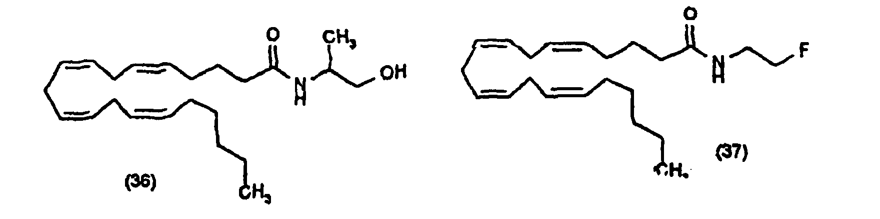

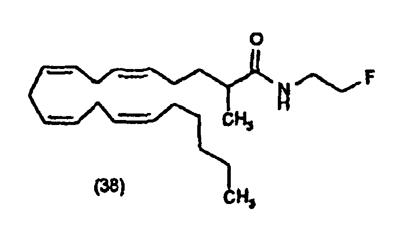

10. Utilisation suivant la revendication 9, dans laquelle l'agoniste du récepteur cannabinoïde CB1 est choisi entre des composés de formules suivantes :
